(19)

Europäisches
Patentamt
European
Patent Office
Office européen
des brevets

(11) **EP 1 797 916 A1**

(12) **EUROPEAN PATENT APPLICATION**
published in accordance with Art. 158(3) EPC

(43) Date of publication:
**20.06.2007 Bulletin 2007/25**

(51) Int Cl.:
***A61M 1/18*** (2006.01)      ***B01D 69/08*** (2006.01)
***B01D 71/68*** (2006.01)

(21) Application number: **05770443.9**

(22) Date of filing: **09.08.2005**

(86) International application number:
**PCT/JP2005/014568**

(87) International publication number:
**WO 2006/016575 (16.02.2006 Gazette 2006/07)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **10.08.2004 JP 2004233446**

(71) Applicants:
• **Toyo Boseki Kabushiki Kaisha**
  **Osaka-shi, Osaka 530-8230 (JP)**
• **NIPRO CORPORATION**
  **Kita-ku, Osaka-shi, Osaka-fu,**
  **531-8510 (JP)**

(72) Inventors:
• **MABUCHI, Kimihiro,**
  **TOYO BOSEKI KABUSHIKI KAISHA**
  **Otsu-shi,**
  **Shiga 5200243 (JP)**
• **YOKOTA, Hideyuki,**
  **TOYO BOSEKI KABUSHIKI KAISHA**
  **Otsu-shi,**
  **Shiga 5200243 (JP)**
• **MONDEN, Noriko,**
  **TOYO BOSEKI KABUSHIKI KAISHA**
  **Otsu-shi,**
  **Shiga 5200243 (JP)**

• **KOYAMA, Shinya,**
  **TOYO BOSEKI KABUSHIKI KAISHA**
  **Otsu-shi,**
  **Shiga 5200243 (JP)**
• **KATO, Noriaki,**
  **TOYO BOSEKI KABUSHIKI KAISHA**
  **Otsu-shi,**
  **Shiga 5200243 (JP)**
• **HATAKEYAMA, Yuuki,**
  **NIPRO CORPORATION**
  **Osaka-shi,**
  **Osaka 5318510 (JP)**
• **SUNOHARA, Takashi,**
  **NIPRO CORPORATION**
  **Osaka-shi,**
  **Osaka 5318510 (JP)**
• **MASUDA, Toshiaki,**
  **NIPRO CORPORATION**
  **Osaka-shi,**
  **Osaka 5318510 (JP)**

(74) Representative: **Vossius & Partner**
**Siebertstrasse 4**
**81675 München (DE)**

(54) **HIGHLY WATER PERMEABLE HOLLOW FIBER MEMBRANE-TYPE BLOOD PURIFIER AND PROCESS FOR PRODUCING THE SAME**

(57)      The present invention relates to a highly water-permeable hollow fiber membrane type blood purifier which comprises hydrophobic polymer hollow fiber membranes each containing a hydrophilic polymer, wherein the hollow fiber membrane has a hydrophilic polymer content of 25 to 50 mass % and a ratio of hole areas of 8 to 25% at its outer surface, and has a thickness non-uniformity degree of 0.6 or more, a thickness of 10 to 60 $\mu$m and a burst pressure of 0.5 to 2 MPa, and which is **characterized in that** the blood purifier has a water permeability of 150 to 2,000 ml/m$^2$/hr/mmHg, and in that said blood purifier is exposed to radioactive rays on conditions that the oxygen concentration of an ambient atmosphere around the hollow fiber membranes is from 0.001% inclusive to 0.1% inclusive, and that the moisture content of the hollow fiber membrane to its weight is from 0.2 mass % inclusive to 7 mass % inclusive. The present invention also relates to a process for manufacturing the same blood purifier.

EP 1 797 916 A1

**Description**

FIELD OF THE INVENTION

The present invention relates to a highly water permeable hollow fiber membrane type blood purifier for medical use, which is excellent in safety and module-assembling ease and which has a high water permeability suitable for use in therapy of chronic renal failure, and also relates to process for manufacturing the same.

BACKGROUND OF THE INVENTION

In the hemocatharsis therapy for renal failure or the like, modules such as hemodialyzers, blood filters and hemodialyzer-filters, using dialysis membranes or ultrafilter membranes as separators are widely used to remove urine toxin and waste products from bloods.
Dialysis membranes and ultrafilter membranes for use as separators are generally formed from natural materials such as cellulose or derivatives thereof (e.g., cellulose diacetate, cellulose triacetate and the like), and synthesized polymers such as polysulfone, polymethyl methacrylate, polyaclyronitrile and the like. Above all, highly importance is attached to modules using hollow fiber type membranes as separators in the field of dialyzers, because of the advantages thereof, such as the reduction of the volume of the extracorporeal-circulated blood, high waste product-removing rate, and high productivity of manufacturing modules.

The main use of a dialysis module using hollow fiber membranes is to remove low molecular weight substances such as urea, creatinine, etc. from blood by generally allowing the blood to flow into the internal voids of hollow fibers while allowing a dialyzate to flow opposing the blood outside the hollow fibers, and allowing the low molecular weight substances to diffuse and transfer from the blood to the dialyzate, thereby removing the low molecular weight substances from the blood. With the increase in the number of patients who undergo therapies of dialysis over long periods of time, the dialysis complications have raised issues, and recently, the subject substances to be removed by dialysis are not only the low molecular weight substances such as urea and creatinine, but also medium molecular weight substances having molecular weights of several thousands and high molecular weight substances having molecular weights of 10,000 to 20,000. Under such a circumstance, hemocatharsis membranes are demanded to remove also these substances. Above all, $\beta 2$ microglobulin having a molecular weight of 11,700 which is known to be a causal substance of carpal tunnel syndrome is a target to be removed. To obtain membranes for use in removal of such high molecular weight substances for therapy, it is preferable to increase the water permeability thereof by increasing the sizes of the holes of the dialysis membranes, increasing the number of the holes thereof, increasing the void content thereof or reducing the thickness thereof.

However, there is a problem in that the improvement of the water permeability induces the elution of more hydrophilic polymers, which leads to the lowered strength of the membranes. The elution of more and more hydrophilic polymers induces side effects and complications since the hydrophilic polymers as foreign matters to human bodies are more and more accumulated in the human bodies over long periods of dialysis therapies. In addition, because of the decreased strengths of the membranes, the fibers thereof are damaged in the course of manufacturing, transporting or handling the same. As a result, the fibers tend to be broken during the therapy to cause the leakage of blood.

As the means for inhibiting the leakage of blood, there is disclosed a technique for seeking proper time during which a spinning dope extruded from a nozzle passes through a gaseous phase, and a proper range of the concentration of a core material, by further decreasing the concentration of an organic solvent in the core material (cf. Patent literature 1). This is a method of forming a thin and dense layer on the inner surface of a membrane while controlling the water permeability of the membrane. However, this method has difficulties in that the water permeability of the membrane is hard to be set within a narrow range, because the dense layer formed on the inner surface of the membrane markedly affects the water permeability of the membrane.

Further, the increasing of the hole sizes, the numbers of the holes or the percentage of void of membranes leads to more contents of hydrophilic polymers on the outer surfaces of the membranes. As a result, there is a high possibility of the infiltration of endotoxin in a dialyzate, into blood to thereby induce side effects such as fever, etc. In another case, hollow fiber membranes stick to one another because of the hydrophilic polymers present on the outer surfaces of the membranes while the membranes are being dried, and therefore, the assembling of a module therefrom becomes hard.

A technique to solve the problem of the endotoxin's infiltration into the blood, among the foregoing problems, is disclosed (cf. Patent Literature 2). This technique takes advantage of the properties of endotoxin which has a hydrophobic moiety in its molecule and thus which tends to be adsorbed to a hydrophobic material. According to this technique, the ratio of a hydrophilic polymer to a hydrophobic polymer on the outer surface of a hollow fiber membrane is 5 to 25 mass %.
While this technique is very preferable as a method of inhibiting the infiltration of endotoxin into blood, it is needed to wash and remove the hydrophilic polymer on the outer surface of the membrane, in order to impart such a property to the membrane. This washing requires long time to treat the membrane, which results in low cost-effectiveness. For example, according to Examples of the above Patent Literature, a membrane is washed by showering hot water of 60°C thereon for one hour, and washed with hot water of 110°C for one hour.

Decreasing the amount of a hydrophilic polymer on the outer surface of a membrane is preferable to inhibit the infiltration of endotoxin into blood. However, the hydrophilicity of the outer surface of the membrane becomes lower, which leads to a lower compatibility of the membrane with a normal saline solution which is used to wet a dried hollow fiber membrane bundle for assembling a module. Accordingly, purging the membrane of an air (priming) in the course of the wetting operation becomes insufficient.

As a method of solving this problem, blending of a hydrophilic compound such as glyceline or the like is disclosed (cf. Patent Literature 3 and 4). However, this method has problems in that the hydrophilic compound acts as a foreign matter during dialysis if the concentration thereof is outside a proper range, and in that the susceptibility of the hydrophilic compound to photodeterioration or the like gives an adverse influence on the storage stability of the module. There is a further problem in that, when a bundle of the hollow fiber membranes is fixed in a module for assembling the same, the bonding of an adhesive is hindered.

To avoid the sticking of the hollow fiber membranes to one another, i.e., another one of the foregoing problems, a method of increasing the ratio of hole areas of the outer surface of a membrane to 25% or more is disclosed (cf. Patent Literature 5). This method is surely preferred to avoid the sticking of the membranes, but has a problem in that the strength of the membrane becomes lower because of the higher ratio of hole areas.

As a result, the leakage of blood as mentioned above occurs. A further disclosed method of avoiding this problem is to specify the ratio of hole areas or the hole area of the outer surface of a membrane (cf. Patent Literature 6). However, this method has a problem in that the water permeability of the membrane becomes lower.

Patent Literature 7 discloses a technique of suppressing, to 10 ppm or less, the elution of a hydrophilic polymer from a hydrophobic polymeric hollow fiber membrane which contains the hydrophilic polymer. However, this technique is accomplished, excluding the consideration of the hemodiafiltration which is demanded to have higher pressure resistance and higher endotoxin-removing performance than the conventional hemodialysis.

For example, this Literature has no teaching about the content of polyvinyl pyrrolidone on the outer surface of a membrane, and the burst pressure, the ratio of hole areas and the average hole area of the outer surface thereof.

Particularly, there is no definite disclosure about the very important factors, i.e., the non-uniform thickness and the burst pressure of the membrane which is attributed to the flaws of the membrane.

Being a medical device, the hollow fiber membrane type blood purifier should be sterilized to prevent the proliferation of bacteria. Incomplete sterilization leads to the above problem attributed to endotoxin, etc. The sterilization treatments using formalin, an ethylene oxide gas, high water vapor pressure, and exposure to radioactive rays such as γ-ray and electron rays are carried out, and each of the methods exhibits its specific effect. Out of these methods, the sterilization method using radioactive rays or electron rays is preferable because this method makes it possible to directly treat a subject matter in a package and is superior in sterilization effect.

However, an adhesive, etc. for use in bonding hollow fiber membranes for a blood purifier tend to deteriorate due to the exposure to radioactive rays or electron rays. Under such a situation, a sterilizing method capable of preventing such deterioration is proposed.

For example, there is known a method for preventing the deterioration of hollow fiber membranes due to the exposure to γ-ray, by maintaining the hollow fiber membranes in a wet state (cf. Patent Literature 8). However, this method has difficulties in that the weight of the blood purifier inevitably becomes larger because of the need of maintaining the hollow fiber membranes in a wet state, which leads to disadvantages in transport and handling, especially in a cold area where water charged in a blood purifier is frozen to burst or damage the hollow fiber membranes in a severely cold season.

There is a further factor of higher cost spent for the preparation of sterilized water, etc. What is worse, the hollow fiber membranes in the wet state facilitate the proliferation of bacteria, and thus it is considered that bacteria are about to proliferate even in a very short time up to the sterilization of the hollow fiber membranes after the packaging thereof. As a result, it takes a long time in perfectly sterilizing a blood purifier made of such hollow fiber membranes, which undesirably leads to higher cost and problems in safety.

As a method for avoiding the wet state of the hollow fiber membranes and inhibiting the deterioration thereof due to the exposure to radioactive rays, it is known that a protecting agent against sterilization, such as glycelin, polyethylene glycol or the like is contained the hollow fiber membtranes, and that the hollow fiber membranes in a dried state are exposed to γ-ray (cf. Patent Literature 9). However, this method also has a problem in that it is difficult to maintain the low moisture content of the hollow fiber membranes since the hollow fiber membranes contain the protecting agent, and arises other problems such as the deterioration of the protecting agent due to the exposure to γ-ray, extra labor and time for washing the hollow fiber membranes to remove the protecting agent just before use, etc.

A known method for solving the preceding problems is to sterilize hollow fiber membranes by lowering the moisture content of the hollow fiber membrane to 5 mass % or less, and exposing the same to radioactive rays under an ambient atmosphere of a relative humidity of 40% or lower (cf. Patent Literature 10). This method is effective to solve the foregoing problems, and by this method, the UV absorbance of the hollow fiber membranes at a wavelength of 220 to 350 nm, measured according to an elution test for a dialytic membrane regulated in the approved standards for manufacturing artificial kidney devices, satisfies the condition of 0.1 or less as a reference value. However, this method is established to avoid the decomposition and deterioration of the protecting agent, etc. only at the sterilizing step, and does not to pay any attention to the long-term storage stability of the hollow fiber membranes.

A further method is disclosed in which hollow fiber membranes whose moistures are kept at 10 mass % or less are exposed to γ-ray to thereby decrease the insolubilized components of the membrane material to 10 mass % or less (cf. Patent Literature 11). This literature teaches that the amount of a hydrophilic polymer per 1 $m^2$ of the target liquid-contacting area of the membrane, extracted from an aqueous 40% ethanol solution, is 2.0 $mg/m^2$ or less. However, the method of Patent Literature 11 is established to avoid the decomposition and deterioration of the adhesive, the protecting agent, etc. only at the sterilizing step, and is not intended to achieve the long-term storage stability of the hollow fiber membranes.

There is known a method for preventing the deterioration of a base material for a medical device due to oxygen. This method comprises the steps of sealing the medical device together with an oxygen scavenger in an oxygen-impermeable packaging material, and exposing the same to radioactive rays, and the application of this method to a blood purifier is also disclosed (cf. Patent Literatures 12, 13 and 14).

The deterioration of the medical device due to the radiation exposure in the presence of the oxygen scavenger is revealed as follows: an odor releases in the method of Patent Literature 12; the strength and dialyzing performance of the base material lower in the method of Patent Literature 13; and the strength of the base material lowers, and aldehydes are formed in the method of Patent Literature 14. However, none of these literature refers to an increase in the amount of the extracted material as described above. Any of theses literatures refers to the oxygen concentration within the package during the radiation exposure, but not to the importance of the moisture of the hollow fiber membranes and the humidity in an atmosphere.

Further, any of these literatures refers to the importance of the gas-, especially, oxygen-impermeability of the base material for the package for use in the method of sterilizing by radiation exposure in a system using the above oxygen scavenger, but not to the humidity-permeability thereof.

Patent Literatures 15 and 16 disclose hollow fiber membrane type blood purifiers any of which uses no filling liquid, and from any of which a hydrophilic polymer is not eluted by replacing the internal atmosphere of the hollow fiber membrane type blood purifier with an inert gas. However, a peroxide, typically a hydrogen peroxide, is formed by radiation exposure, since the oxygen concentration is high at the sterilization step, or since no attention is paid to the importance of the humidity of the ambient atmosphere, and thus, the resulting blood purifier lacks long-term storage stability.

Patent Literature 1: JP-A-2000-107577
Patent Literature 2: JP-A-2000-254222
Patent Literature 3: JP-A-2001-190934
Patent Literature 4: Patent No. 3193262
Patent Literature 5: JP-A-2001-38170
Patent Literature 6: JP-A-2000-140589
Patent Literature 7: JP-A-2001-170171
Patent Literature 8: JP-B-55-23620
Patent Literature 9: JP-A-8-168524
Patent Literature 10: JP-A-2000-288085
Patent Literature 11: JP-A-2001-205057
Patent Literature 12: JP-A-62-74364
Patent Literature 23: JP-A-62-204754
Patent Literature 14: WO98/58842
Patent Literature 15: JP-A-2001-170167
Patent Literature 16: JP-A-2003-245526

DISCLOSURE OF THE INVENTION

PROBLEM TO BE SOLVED BY THE INVENTION

Objects of the present invention are to provide a medical hollow fiber membrane type blood purifier having high water permeability, which is excellent in safety and module-assembling ease and is suitable for therapy of chronic renal failure, and to provide a process for manufacturing the same.

MEANS FOR SOLVING THE PROBLEM

The present invention relates to a highly water permeable hollow fiber membrane type blood purifier which include therein hydrophobic polymeric hollow fiber membranes each containing a hydrophilic polymer, and which is characterized in that the content of the hydrophilic polymer in the outer surface of the hollow fiber membrane is from 25 to 50 mass %; the percentage of hole areas of the outer surface of the same is from 8 to 25%; the non-uniformity in thickness of the same is 0.6 or more; the thickness of the same is from 10 to 60 $\mu$m; the burst pressure of the same is from 0.5 to 2 MPa; and the water permeability of the blood purifier is from 150 to 200 ml/m$^2$/mmHg, and which is characterized in that the hollow fiber membrane whose moisture is maintained at 0.2 to 7 mass % to its weight is exposed to radioactive rays under an ambient atmosphere having an oxygen concentration of from 0.001 to 0.1%.
The present invention also relates to a process for manufacturing a highly water permeable hollow fiber membrane type blood purifier, which includes a step of exposing the hollow fiber membranes to radioactive rays as described above.

EFFECT OF THE INVENTION

The highly water permeable hollow fiber membrane type blood purifier of the present invention is suitable for use as a medical hollow fiber type blood purifier which is excellent in safety and module-assembling ease and which has high water permeability and is suitable for therapy of chronic renal failure.

BEST MODES FOR CARRYING OUT THE INVENTION

The hollow fiber membrane to be used in the present invention comprises a hydrophobic polymer containing a hydrophilic polymer moiety. Examples of a material for the hydrophobic polymer of the present invention include cellulose resins such as regenerated cellulose, cellulose acetate and cellulose triacetate; polysulfone resins such as polysulfone and polyether sulfone; polyacrylonitrile; polymethyl methacrylate; ethylene vinyl-alcohol copolymers; and the like. Above all, the cellulose resins and the polysulfone resins are preferable, since the use of these resins makes it easy to obtain hollow fibers having a coefficient of water permeability of 150 mL/m$^2$/hr./mmHg or more. More preferable are cellulose diacetate and cellulose triacetate among the cellulose resins, and polyether sulfone among the polysulfone resins, since the use of such resins makes it easy to reduce the thickness of membranes.
Although not particularly limited, the hydrophilic resin to be preferably used in the present invention is such one that can form a micro phase-separated structure with the hydrophobic polymer in a solution. Specific examples of the hydrophilic polymer include polyethylene glycol, polyvinyl alcohol, carboxylmethyl cellulose, polyvinyl pyrrolidone and the like. The use of polyvinyl pyrrolidone is preferred in view of safety and cost-effectiveness.
In the present invention, the content of the hydrophilic polymer to the hydrophobic polymer in the membrane is within such a range that is enough to impart sufficient hydrophilicity and high moisture to the hollow fiber membrane. Preferably, the content of the hydrophobic polymer is 80 to 99 mass %, and that of the hydrophilic polymer, 1 to 20 mass %. When the content of the hydrophilic polymer to the hydrophobic polymer is too low, the hydrophilicity-imparting effect to the membrane may be poor. Therefore, the content of the hydrophilic polymer is preferably 2 mass % or more. On the other hand, when the above content is too high, the hydrophilicity-imparting effect saturates, and the amount of the hydrophilic polymer eluted from the membrane tends to increase, and may exceed 10 ppm as will be described later. Therefore, the content of the hydrophilic polymer is more preferably 18 mass % or less, still more preferably 15 mass % or less, particularly 12 mass % or less, most preferably 9 mass % or less.
In the present invention, the amount of the hydrophilic polymer eluted from the hollow fiber membrane is preferably 10 ppm or less. When this amount exceeds 10 ppm, a side effect or a complication may be induced due to the eluted hydrophilic polymer if a patient undergoes a dialysis therapy over a long period of time. There is no limit in selection of a method of satisfying the above properties. For example, these properties can be obtained by restricting the content of the hydrophilic polymer to the hydrophobic polymer to the above specified range, or by optimizing the film-forming conditions for the hollow fiber membrane. The amount of the hydrophilic polymer eluted from the membrane is more preferably 8 ppm or less, still more preferably 6 ppm or less, particularly 4 ppm or less. This amount is ideally zero in view of safety to human bodies. However, when the amount of the hydrophilic polymer eluted from the membrane is zero, the hydrophilicity of the surface of the membrane in contact with the blood becomes lower so that the compatibility of the membrane to the blood may be poor. Therefore, about 0.1 ppm or so of the hydrophilic polymer eluted from the membrane is allowable.
In one of the preferred modes of the present invention, the hydrophilic polymer is crosslinked to be insoluble. The method of crosslinking, the degree of crosslinking, etc. are not limited. Crosslinking by $\gamma$-rays, electron rays or heat, chemical crosslinking or the like may be employed. Particularly, crosslinking by $\gamma$-rays or electron rays is preferable, because any residue such as an initiator does not remain and because the penetration degree of $\gamma$-rays or electron rays into the material is high. In the present invention, preferably, a module is charged with a degassed aqueous RO solution at high density and sealed, and is then exposed to 10 to 60 kGY of γ-rays. When the exposure amount of γ-rays is too small, the crosslinking is insufficient to increase the amount of the eluted components. Therefore, it is preferable to expose the module to γ-rays to an absorbed amount of 15 kGy or more. When the exposure amount of γ-rays is too large, the hydrophobic polymer, the hydrophilic polymer, the housing and an urethane resin may disintegrate and deteriorate. Thus, the exposure amount of γ-rays is preferably 50 kGy or less, more preferably 40 kGy or less, particularly 30 kGy or less. The degassed aqueous RO solution herein referred to means an aqueous RO solution which is obtained by heating the solution to a temperature of a room temperature to 50°C, and stirring the solution for 15 mins. to 2 hours while decompressing the same to -500 to -750 mmHg. When the solution, not degassed, is used, the oxygen dissolved in water oxidizes and deteriorates the components of the membrane, and consequently, the eluted components tend to increase.

In the present invention, the insolubilization is confirmed based on the solubility of the crosslinked membranes found when the same membranes are dipped in dimethylformamide. That is, 1.0 g of the crosslinked membranes are cut out and then are dipped in 100 ml of dimethylformamide so as to visually observe the presence of the insoluble components. In case of a module charged with a liquid, firstly, the liquid is removed; then, pure water is allowed to flow into a passage on the side of a dialyzate at a rate of 500 mL/min. for 5 minutes; and then, pure water is similarly allowed to flow into a passage on the side of blood at a rate of 200 mL/min. for 5 minutes. Finally, pure water is allowed to pass through the membranes from the side of the blood to the side of the dialyzate at a rate of 200 mL/min. Thus, the washing of the module is completed. The hollow fiber membranes are removed from the resultant module, and then are freeze-dried for use as a sample which is to be used to measure insoluble components. Also, a module comprising dried hollow fiber membranes is similarly washed so as to be used as a sample.

In the present invention, the content of the hydrophilic polymer in the outer surface of the hollow fiber membrane is 25 to 50 mass %. When the content of the hydrophilic polymer in the outer surface of the membrane is too low, the content of the hydrophilic polymer in a whole of the membrane, particularly in the inner surface of the membrane becomes too low, which may lower the compatibility of the membrane with blood or the permeability of the membrane. When the dried membranes are used, the priming performance thereof tends to be low. When a hemodialyzer is used for hemocatharsis, it is needed to wet and degas the hollow fiber membranes by allowing physiological saline or the like to pass through the outer and inner portions of the hollow fiber membranes. In this priming operation, it is considered that the roundness of the hollow fiber membranes, the crushing of the end portions thereof, the deformation thereof, the hydrophilicity of the material for the membranes and so on may give some influence on the priming performance of the membranes. When a module comprising dried hollow fiber membranes formed of a hydrophobic polymer and a hydrophilic polymer is used, the balance between the hydrophilic polymer and the hydrophobic polymer in the hollow fiber membrane gives serious influence on the priming performance of the membranes. Accordingly, the content of the hydrophilic polymer is more preferably 27 mass % or more, still more preferably 30 mass % or more. When the content of the hydrophilic polymer in the outer surface of the membrane is too high, the possibility of the infiltration of endotoxin from a dialyzate into the side of blood becomes higher to induce side effects such as fever; or the hollow fiber membranes stick to one another due to the hydrophilic polymer on the outer surfaces of the membranes, when the membranes are dried, so that the assembling of the module becomes hard. Therefore, the content of the hydrophilic polymer is more preferably 47 mass % or less, still more preferably 45 mass % or less.

To control the content of the hydrophilic polymer in the outer surface of the hollow fiber membrane within the above specified range, for example, the content of the hydrophilic polymer to the hydrophobic polymer is controlled within the above specified range; or otherwise, the conditions for forming the hollow fiber membranes are optimised. It is also an effective method to wash the hollow fiber membranes manufactured. In the manufacturing of the hollow fiber membranes, the humidity of the air gap portion of the outlet of a nozzle is adjusted; and the drawing conditions, the temperature of a solidifying bath, the composition ratio of a solvent to a non-solvent in a solidifying liquid, etc. are optimally controlled. The membranes are effectively washed with hot water or alcohol, or by centrifugation.

Preferably, the air gap portion is enclosed with a material capable of shielding the air gap from an external air. Preferably, the humidity of the inside of the air gap portion is controlled by the composition of a spinning dope, the temperature of the nozzle, the length of the air gap, the temperature of the external solidifying bath and the composition of the liquid. For example, a spinning dope of the following composition is extruded from a nozzle heated to a temperature of 30 to 60°C, and the resultant semi-solid fibers are allowed to pass through the air gap with a length of 100 to 1,000 mm, and are then introduced into an external solidifying bath with a concentration of 0 to 70 mass % and of a temperature of 50 to 80°C, wherein the composition of the spinning dope is as follows: polyethersulfone/polyvinyl pyrrolidone/dimethylacetoamide/ RO water = 10 to 25/0.5 to 12.5/52.5 to 89.5/0 to 10.0. In this case, the absolute humidity of the air gap portion induces a dried air (0.01 to 0.3 kg/kg). By controlling the humidity of the air gap portion within the above specified range, the ratio of hole areas, the average hole area and the content of the hydrophilic polymer of the outer surface of the membrane can be controlled within proper ranges, respectively.

As the internal solidifying liquid, an aqueous solution of 0 to 80 mass % of dimethylacetoamide (DMAc) is preferred. When the concentration of the internal solidifying liquid is too low, the dense layer of the inner surface of the hollow fiber membrane becomes thick, which leads to a lower solute permeability. The concentration of the internal solidifying liquid is more preferably 15 mass % or more, still more preferably 25 mass % or more, particularly 30 mass % or more. When this concentration is too high, the dense layer tends to be formed incompletely, and thus tends to have a lower fractional property. Accordingly, the concentration of the internal solidifying liquid is more preferably 70 mass % or less, still more preferably 60 mass % or less, particularly 50 mass % or less.

As the external solidifying liquid, an aqueous solution of 0 to 50 mass % of DMAc is preferably used. When the concentration of the external solidifying liquid is too high, the ratio of hole areas and the average hole area of the outer surface of the membrane become too large, which may induce more possibility of the back flow of endotoxin into the side of blood during a dialysis, or the decrease of the burst pressure. Accordingly, the concentration of the external solidifying liquid is more preferably 40 mass % or less, still more preferably 30 mass % or less, particularly 25 mass % or less. When this concentration is too low, it is needed to use a large amount of water to dilute the solvent brought from the spinning dope, and the cost for treating the waste liquid becomes higher. Therefore, the lower limit of the concentration of the external solidifying liquid is more preferably 3 mass % or more, still more preferably 5 mass % or more.

In manufacturing the hollow fiber membranes of the present invention, preferably, the membranes are not substantially drawn before the structures of the hollow fiber membranes are completely fixed. The wording "the membranes are not substantially drawn" means that the speeds of rolls are controlled in the spinning step so that the spinning dope extruded from the nozzle is not loosen or excessively pulled. The ratio of the linear speed of extrusion to the speed of the first roll in the solidifying bath (draft ratio) is preferably 0.7 to 1.8. When the draft ratio is too small, the hollow fiber membranes being fed are loosen, which leads to lower productivity. Therefore, the draft ratio is more preferably 0.8 or more, still more preferably 0.9 or more, particularly 0.95 or more. When the draft ratio is too large, the dense layers of the hollow fiber membranes tear and thus, the structures of the membranes may be destructed. Therefore, the draft ratio is more preferably 1.7 or less, still more preferably 1.6 or less, particularly 1.5 or less, most preferably 1.4 or less. By controlling the draft ratio within this range, the deformation or destruction of the holes of the membranes can be prevented, and thus, the holes of the membranes are not clogged with the protein in blood. As a result, the membranes can exhibit performance stability and sharp fractional properties over a long period of time.

The hollow fiber membranes having passed through a water bath are wound onto a hank in a wet state, to thereby form a bundle of 3,000 to 20,000 membranes. The resultant bundle of hollow fiber membranes is washed to remove the excessive solvent and hydrophilic polymer. In the present invention, the bundle of hollow fiber membranes is washed by dipping the same in hot water of 70 to 130°C or an aqueous solution of 10 to 40 vol.% of ethanol or isopropanol of a room temperature to 50°C. Also, the following washing methods are preferred.

(1) In case of washing with hot water, the bundle of hollow fiber membranes is dipped in excessive RO water and treated therein at a temperature of 70 to 90°C for 15 to 60 minutes. Then, the bundle thereof is removed and subjected to centrifugal hydroextraction. This operation is repeated three or four times for washing, while the RO water is replaced.

(2) Otherwise, the bundle of hollow fiber membranes dipped in excessive RO water in a compressed container may be treated at 121°C for about 2 hours.

(3) The same operation as the above operation (1) is repeated, also when an aqueous solution of ethanol or isopropanol is used.

(4) The bundle of hollow fiber membranes is radially arranged in a centrifugal washing machine, and is then subjected to centrifugal washing for 30 minutes to 5 hours in total, while a washing liquid of 40 to 90°C is being shower-like sprayed onto the bundle from the center of rotation.

Two or more of the above methods may be combined. When the treating temperature is too low in any of the above methods, it is needed to increase the number of washing, which results in higher cost. When the treating temperature is too high, the decomposition of the hydrophilic polymer is accelerated, which, on the contrary, lowers the washing efficiency. By this washing, it becomes possible to properly adjust the content of the hydrophilic polymer in the outer surfaces of the membranes and to inhibit the sticking of the membranes or to decrease the amount of the eluted components.

In this regard, the content of the hydrophilic polymer in the outermost surface of the hollow fiber membrane is measured and calculated by the ESCA method as will be described later: that is, the absolute value of the content of the same polymer in the outermost surface of the hollow fiber membrane (with a depth of several to several tens Å from the surface layer) is determined. Generally, the ESCA method makes it possible to measure the content of the hydrophilic polymer (PVP) in the surface (the outermost layer) of the hollow fiber membrane, up to a depth of about 10 nm (100 Å) from the surface thereof.

Another feature of the present invention is that the burst pressure of the hollow fiber membranes set in the blood purifier is 0.5 MPa or higher, and the coefficient of water permeability of the blood purifier, 150 m1/m2/hr./mrnHg or higher. When the burst pressure is too low, it becomes impossible to detect a latent defect which leads to the leakage of blood as will be described later. When the coefficient of water permeability is too low, the dialysis efficiency becomes lower. It is effective to increase the hole size or increase the number of holes of the membrane, in order to improve the dialysis efficiency. However, this method has a problem in that the strength of the membranes decreases or in that the membranes have defects. In contrast, the hollow fiber membranes of the present invention have strength balanced with the resistance to the permeation of a solute, by optimizing the hole sizes of the outer surfaces of the membranes to thereby optimize the percentage of voids of the support layers. The coefficient of water permeability is more preferably 200 ml/m$^2$/hr./mmHg or more, still more preferably 300 ml/m$^2$/hr./mmHg or more, particularly 400 ml/m$^2$/hr./mmHg or more, and most preferably 500 ml/m$^2$/hr./mmHg or more. When the coefficient of water permeability is too high, the water-removing control during hemodialysis becomes difficult. Therefore, the coefficient of water permeability is preferably 2,000 ml/m$^2$/hr./mmHg or less, more preferably 1,800 ml/m$^2$/hr./mmHg or less, still more preferably 1,500 ml/m$^2$/hr./mmHg or less, particularly 1,300 ml/m$^2$/hr./mmHg or less, and most preferably 1,000 ml/m$^2$/hr./mmHg or less.

The present inventors have studied the physical properties of hollow fiber membranes suitable for use in a blood purifier. Generally, a blood purifier is subjected to a leak test by compressing the internal and external portions of the hollow fiber membranes with an air, in the final stage for providing a product, so as to check the defects of the hollow fiber membrane and a module. When any leakage is detected by the compressed air, the module is scrapped as a defective, or such defects of the module are repaired. The air pressure for use in this leak test is often several times larger than the proof pressure (generally 500 mmHg) for a hemodialyzer. In this regard, the present inventors have found that the very fine flaws, crushing or tearing of hollow fiber membranes having very high water permeability, which can not be detected by any of conventional compression leak tests, cause the cutting or pin holes of the hollow fiber membranes, in the course of the manufacturing steps after the leak test (mainly in the step of sterilization or packing), in the course of transporting, or in the course of handling in a clinical site (unpacking or priming); and the present inventors also have found that such cutting or pin holes in the membranes cause troubles such as the leakage of blood during a therapy. As a result of the inventors' intensive researches about this problem, it is found that the pressure of the compressed air for use in the conventional leak tests is insufficient to detect such latent defects of hollow fibers that lead to the occurrence of cutting or pin holes of the hollow fiber membranes used in a clinical site. Then, the present inventors have found that a still higher pressure is needed to detect such latent defects, and that it is effective to prevent the occurrence of the non-uniform thickness of the hollow fiber membranes, in order to prevent the occurrence of the above latent defects. The present invention is accomplished based on such findings.

The burst pressure referred to in the present invention is used as an index for the pressure-resisting performance of a module made up of the hollow fiber membranes: the burst pressure is a pressure which bursts the hollow fiber membranes, when the interiors of the hollow fiber membranes are compressed with a gas while the applied pressure is being gradually increased until the hollow fiber membranes can not withstand their internal pressures and are finally burst. The burst pressure is preferably 0.5 MPa or higher, more preferably 0.55 Mpa or higher, particularly 0.6 MPa or higher, since the possibility of causing cutting or pin holes in the hollow fiber membranes in use becomes lower, as the burst pressure becomes higher. When the burst pressure is lower than 0.5 MPa, the hollow fiber membranes may have possible latent defects. Although a higher and higher burst pressure is desirable, intended membrane performance can not be obtained, when the thickness of the membranes is increased or when the void ratio is excessively decreased, in order to increase the burst pressure. When hemodialysis membranes are intended, the burst pressure of the membranes is preferably 2.0 MPa or lower, more preferably 1.7 MPa or lower, still more preferably 1.5 MPa or lower, still more preferably 1.3 MPa or lower, particularly 1.0 MPa or lower.

The present invention is accomplished also based on the finding that the safety of the conventional hollow fiber membranes can not be reliably ensured over a long period of dialysis therapy because of the blood leakage therefrom which occurs depending on the macro properties such as the strength of the membranes or the like. To ensure the safety of the membranes against the blood leakage over a long period of dialysis therapy, the present inventors have intensively studied in order to establish an evaluation method for detecting the above-described latent defects in addition to the macro properties of membranes. As a result of such efforts, the present invention is accomplished.

The non-uniformity herein referred to in the present invention means the non-uniform thickness of the partial sections of 100 hollow fiber membranes in a blood purifier, when the sections of the hollow fiber membranes are observed. The non-uniformity is indicated by a ratio of a maximum value to a minimum value. The present invention is characterized in that the minimum non-uniformity found from 100 hollow fibers is 0.6 or more. When even only one hollow fiber having a non-uniformity of smaller than 0.6 is included in 100 hollow fibers, such a hollow fiber has a danger of causing the leakage of blood during a clinical therapy. Therefore, the non-uniformity referred to in the present invention is not an average value but a minimum value among the values of non-uniformity of the 100 hollow fibers. The higher the non-uniformity, the better it is, because the uniformity of the membranes is improved so that the actual development of latent defects of the membranes is suppressed to thereby increase the burst pressure. The non-uniformity is more preferably 0.7 or more, still more preferably 0.8 or more, particularly 0.85 or more. When the non-uniformity is too low, the latent defects of the membranes tend to occur as actual defects, so that the burst pressure becomes lower and thus, the leakage of blood from the membranes tends to easily occur.

The thickness of the hollow fiber membranes is preferably 10 to 60 $\mu$m. When this thickness is too large, the permeability of a polymeric substance which has a medium or high molecular weight and which transfers at a low speed becomes lower, although the water permeability of the membranes is high. As the membranes become thinner and thinner, the substance-permeability becomes higher. Thus, the thickness of the membrane is more preferably 55 $\mu$m or less, still more preferably 50 $\mu$m or less, particularly 47 $\mu$m or less. When the thickness of the membranes is too small, the strength of the membranes is low, and the burst pressure becomes lower even if the non-uniformity is 0.6 or more. Therefore, the thickness of the membranes is more preferably 20 $\mu$m or more, still more preferably 25 $\mu$m or more, particularly 30 $\mu$m or more, most preferably 35 $\mu$m or more.

The hollow fiber membranes of the present invention are suitable for use as hollow fiber membranes for hemocatharsis, and particularly suitable for use as hollow fiber membranes for the therapy of renal failure, such as hemodialysis, hemodiafiltration, hemofiltration or the like.

The hollow fiber membranes for use in a blood purifier are preferably manufactured by a dry process using a solution which is prepared by dissolving, in a solvent, the blend of a hydrophobic polymer and a hydrophilic polymer at the foregoing composition ratio. As mentioned above, it is effective to control the non-uniformity of the hollow fiber membranes to 0.6 or more in order to increase the burst pressure to 0.5 MPa or higher. To control the non-uniformity of the membrane to 0.6 or more, for example, it is preferable to strictly uniform the width of the slit of a nozzle which is an outlet for extruding the membrane-forming solution. Generally used as a spinning nozzle for hollow fiber membranes is a tube-in-orifice type nozzle having an annular portion for extruding a spinning dope, and a hole for extruding a core solution which is a hollow portion-forming agent, inside the annular portion. The width of the slit indicates the width of the outer annular portion for extruding the spinning dope. By reducing the variation in the width of the slit, the non-uniformity of the thickness of spun hollow fiber membranes can be decreased. Specifically, the ratio of a maximum value to a minimum value of the width of the slit is controlled to 1.00 to 1.11, and preferably, the difference between the maximum value and the minimum value is adjusted to preferably 10 $\mu$m or less, more preferably 7 $\mu$m or less, still more preferably 5 $\mu$m or less, particularly 3 $\mu$m or less. In addition, the temperature of the nozzle is optimized: the temperature of the nozzle is preferably 20 to 100°C. When the temperature of the nozzle is too low, the nozzle is susceptible to the influence of a room temperature, and the temperature thereof is not steady, so that extrusion spots of the spinning dope often occur. Accordingly, the temperature of the nozzle is more preferably 30°C or higher, still more preferably 35°C or higher, far more preferably 40°C or higher. When the temperature of the nozzle is too high, the viscosity of the spinning dope excessively lowers, so that the steady extrusion of the spinning dope is impossible, and that the thermal deterioration or decomposition of the hydrophilic polymer may proceed. Therefore, the temperature of the nozzle is more preferably 90°C or lower, still more preferably 80°C or lower, particularly 70°C or lower.

To further increase the burst pressure, the flaws of the surfaces of the hollow fiber membranes and the inclusion of foreign bodies and bubbles are lessened to thereby decrease the latent defects of the membranes. To prevent the occurrence of flaws on the membranes, it is effective to optimize the materials for rollers and guides for use in the steps of manufacturing hollow fiber membranes, and to optimize the roughness of the surfaces thereof. It is also effective to decrease the number of times of contact between a module casing and the bundle of hollow fiber membranes or the number of frictions between each of the hollow fiber membranes, when the bundle of the hollow fiber membranes is set in the module casing to make up the module. In the present invention, the rollers to be used is preferably planished at their surfaces in order to prevent the hollow fiber membranes from slipping and having flaws on the surfaces thereof. The surfaces of the guides to be used are preferably matte-finished or knurly finished to avoid the contact with the hollow fiber membranes as much as possible. The bundle of hollow fiber membranes is not directly inserted into the module casing, but preferably, the bundle of hollow fiber membranes wrapped in a matte-firished film is inserted into the module casing, and then, only the film is removed from the module casing.

To prevent the hollow fiber membranes from including foreign bodies, it is effective to use materials containing less foreign bodies, or to decrease the amount of foreign bodies by filtering the spinning dope for forming the membranes. In the present invention, the spinning dope is filtered through a filter having holes with diameters smaller than the thickness of the hollow fiber membranes. Specifically, the spinning dope which is homogeneously dissolved is allowed to pass through a sintered filter which has holes with diameters of 10 to 50 $\mu$m and which is located on a passage along which the spinning dope is guided from a dissolution tank to a nozzle. The filtering may be done at least once, however, it is preferable to make the filtering treatment in a plurality of steps in order to improve the filtering efficiency and to prolong the life of the filter. The diameter of the holes of the filter is preferably 10 to 45 $\mu$m, more preferably 10 to 40 $\mu$m, still more preferably 10 to 35 $\mu$m. When the diameter of the holes of the filter is too small, the back pressure increases, and the quantitative evaluation of the extruded spinning solution degrades.

To prevent the inclusion of bubbles in the membranes, it is effective to degass the polymer solution for forming membranes. Stationary degassing or decompression degassing may be employed in accordance with the viscosity of the spinning dope. In concrete, the inner space of a dissolution tank is decompressed to -100 to -750 mmHg, and then is sealed, and the tank is left to stand in a still state for 5 to 30 minutes. This operation is repeated several times for degassing the tank. When the decompression degree is too low, it is needed to increase the number of times of degassing, which requires longer time. When the decompression degree is too high, high cost is often required to improve the sealing degree of the system. The total time for the degassing treatment is preferably 5 minutes to 5 hours. When the treating time is too long, the hydrophilic polymer may be decomposed and deteriorated due to the decompression effect. When the treating time is too short, the effect of degassing becomes poor.

In the present invention, preferably, the ratio of hole areas of the outer surface of the hollow fiber membrane is 8 to 25%, and the average hole area of the hole area portion of the outer surface of the hollow fiber membrane is 0.3 to 1.0 $\mu m^2$, in order to impart the foregoing features to the membrane. When the ratio of hole areas and the average hole area are too small, the water permeability tend to lower. When the hollow fiber membranes are dried, the membranes are stuck to one another due to the hydrophilic polymer present on the outer surfaces of the membranes, which makes it hard to assemble the module. Therefore, the ratio of hole areas is more preferably 9% or more, still more preferably 10% or more. The average hole area is more preferably 0.4 $\mu m^2$ or more, still more preferably 0.5 $\mu m^2$ or more, far more preferably 0.6 $\mu m^2$ or more. On the contrary, when the ratio of hole areas and the average hole area are too large, the burst pressure tends to lower. Therefore, the ratio of hole areas is more preferably 23% or less, still more preferably 20% or less, particularly 17% or less, most preferably 15% or less. The average hole area is more preferably 0.95 $\mu m^2$ or less, still more preferably 0.90 $\mu m^2$ or less.

To control the content of the hydrophilic polymer to the hydrophobic polymer in the membrane within the above-specified range, for example, the composition ratio of the hydrophobic polymer to the hydrophilic polymer in the spinning dope is adjusted to 95: 5 to 67: 33; the condition for the external solidifying liquid is adjusted to 5 to 40 mass %; or the resultant membranes are washed with hot water or alcohol.

The present invention is accomplished by the present inventors' intensive researches for optimizing the content of the hydrophilic polymer in the outer surface of the hollow fiber membrane and for optimizing the burst pressure, as separate techniques. Surprisingly, they have found that an unexpected synergetic effect as will be described later is produced by simultaneously carrying out both the techniques which appear to be independently of each other. The present invention is achieved based on such a finding. Recently, hemodiafiltration has attracted public attentions in the hemodialysis therapy. The hemodiafiltration is invented in order to remove even low molecular weight proteins, by adding the effect of filtration to the conventional hemodialysis which is conducted mainly by making use of the effect of diffusion. In the hemodiafiltration, forced liquid replacement between blood and a dialyzate is conducted by inducing a larger difference in pressure between the blood and the dialyzate under a pump load. Therefore, the hollow fiber membranes are required to have pressure resistance which has never been required for the conventional membranes. The latent defects of membranes which have not been considered so serious, therefore, may raise actual problems in the hemodiafiltration. The present inventors have discovered that the defects of membranes can be previously detected by increasing the burst pressure to a given value or more, so as to ensure the safety of the membranes as commercial products capable of sufficiently corresponding to the hemodiafiltration. In the hemodiafiltration, the liquid replacement between large amounts of blood and a dialyzate is conducted as mentioned above. In the blood inlet portion of the module, forward filtering proceeds in the direction from blood to a dialyzate, and in the blood outlet portion of the module, the dialyzate is caused to flow back because of backward filtration in the direction from the dialyzate to the blood. When endotoxin, etc. in the eluted component derived from the material of the hollow fiber membranes and the dialyzate are mixed into the blood during the therapy, there is a danger of inducing grave symptoms such as anaphylaxis, etc. In the hollow fiber membrane of the present invention, the amount of the hydrophilic polymer in the outer surface of the membrane is controlled within the specified range and the ratio of hole areas and the hole area of the surface of the membrane are controlled within the specified ranges. By doing so, no leakage of blood from the membrane occurs during the hemofiltration or the hemodiafiltration, even if the hollow fiber membrane has a high water permeability (having holes with large diameters and a high void ratio). Thus, the solute-removing performance for preventing the inclusion of foreign bodies into blood and the safety can be concurrently achieved at high levels.

As a result of the present inventors' extensive efforts, the hollow fiber membranes having the above-described features can be obtained. However, these hollow fiber membranes show extremely decreased fiber strength when a blood purifier comprising the same hollow fiber membranes is sterilized by exposure to radioactive rays. Consequently, the eluted components from the membranes increase in amount, and thus, it is found that the long-term storage stability of the membranes still has a problem to be solved.

The observation by gel permeation chromatography has revealed that this decrease in fiber strength attributed to the sterilization by exposure to radioactive rays is caused by the scission of the main chains of the polymers constituting the hollow fiber membrane. Further deliberate examination thereof suggests that the oxygen concentration during the sterilization by radioactive rays gives serious influence on a blood purifier comprising the hollow fiber membranes. Under an atmosphere with a high oxygen concentration, the main chains of both the hydrophobic polymer and the hydrophilic polymer constituting the hollow fiber membrane are scissored. The use of an inert gas makes it possible to decrease the oxygen concentration to 10% or less to thereby inhibit the scission of the main chain of the hydrophobic polymer, but not the main chain of the hydrophilic polymer. It is found that, by further decreasing the oxygen concentration to 0.1% or less, the scission of the main chain of the hydrophilic polymer also can be inhibited, and simultaneously that the eluted components from the hollow fiber membrane type blood purifier after the sterilization can be reduced in amount. Herein, the eluted component is measured from an UV absorbance regulated in the approved standards for manufacturing artificial kidney devices, and by an elution test for a circuit according to the approved standards for manufacturing artificial kidney devices, and by the measurement of the concentration of hydrogen peroxide representing peroxides, so as to confirm the long-term storage stability of the membranes. As a result, it is found that, when the oxygen concentration is decreased to 0.1% or less, the decrease of the fiber strength attributed to the scission of the main chains of the polymers and the decrease of the burst pressure can be solved, and further that all the items of the tests according to the approved standards for manufacturing artificial kidney devices can be cleared. However, there still remains a problem to be solved: that is, the amount of eluted hydrogen peroxide is not stabilized. As a result of further investigation, it is found that, as one of possible methods, further decreasing of the oxygen concentration is effective to decrease the amount of peroxides. Thus, it is known as a preferable method to further decrease the oxygen concentration. To decrease the oxygen concentration, oxygen is successfully replaced with an inert gas such as argon, nitrogen or the like. If there is a limit in this method, the oxygen concentration can be decreased by using an oxygen scavenger in a sealed system. This method requires a longer time, since a chemical reaction is involved in the reaction of oxygen with the oxygen scavenger within the system after adding the oxygen scavenger in the system. The chemical reaction rapidly proceeds in a stochastic manner, when the oxygen concentration in the system is high, but the chemical reaction becomes slow in a stochastic manner after the oxygen concentration has been decreased. To achieve the oxygen concentration proposed in the present invention, the system is left to stand at a room temperature for 10 hour or longer, preferably 18 hours or longer, more preferably 24 hours or longer. In view of the productivity and the proliferation of bacteria, it is preferable to expose the blood purifier to radioactive rays within 72 hours. It is preferable to decrease the oxygen concentration to an extreme, in order to decrease the amount of peroxides. However, it is found that, by selecting the deoxidation time in order to improve the production efficiency and to reduce the production cost, and then adjusting the oxygen concentration to 0.001% or more, the level of the peroxide can be stabilized. The oxygen concentration is preferably from 0.001 to 0.1%, more preferably from 0.003 to 0.05%, in view of the stability of the eluted component, when the blood purifier is exposed to radioactive rays.

To reduce the amount of the peroxide which is formed during the sterilization by radiation exposure, it is found that it is effective to eliminate the influence of moisture in the hollow fiber membranes and/or an ambient atmosphere, in addition to the reduction of the oxygen concentration. Firstly, the moisture in the hollow fiber membrane is preferably 7 mass % or less, to ensure the adhesion with a seal resin such as an urethane resin which is used to make up a blood purifier. The moisture in the hollow fiber membrane is more preferably 6 mass % or less, still more preferably 5 mass % or less, to ensure the productivity. Under an atmosphere of normal temperature and normal pressure where the hollow fiber membranes can be actually observed, the equilibrium moisture regain of the membranes is around 2 mass %. In the moisture region above 2 mass %, the formation of peroxide can be inhibited, because the moisture content of the hollow fiber membrane is supposed to inhibit the formation of free radicals. Although it is possible to control the dry final moisture to from 2 to 7 mass % by controlling the weight, to thereby ensure, the adhesivity and to inhibit the formation of peroxide, some problems are still left to be solved in view of improvement of the productivity. Under such a situation, the present inventors have further intensively investigated. As a result, it is found that, when the hollow fiber membrane are exposed to radioactive rays, an ambient atmosphere around the hollow fiber membrane, having a relative humidity of higher than 40%RH at 25°C, is effective to reduce the amount of peroxides, even though the moisture content of the hollow fiber membrane itself is less than 2 mass %. Although the action or mechanism thereof is not definitely known, it is supposed that the moisture in an air reacts with radioactive rays to thereby prevent the radioactive rays from directly attacking the polymers constituting the membrane, so that the amount of the peroxide can be decreased. It is possible to suppose that the moisture content of the membrane being sterilized by radiation exposure may act as a buffer against the attacking by the free radicals formed by the radiation exposure. By making effective use of the above conditions, the amount of the peroxide can be reliably reduced, even when the moisture content of the hollow fiber membrane is less than 2 mass %. Therefore, the sterilization is supposed to be possible when the moisture content of the membrane is substantially 0 mass %. However, in view of a substantial dry state, the moisture content of the membrane is preferably 0.2 mass % or more. In view of the improvement of the productivity and the reduction of the running cost, the moisture content of the membrane is more preferably 0.5 mass % or more, still more preferably 1 mass % or more.

When the ambient atmosphere around the hollow fiber membrane during the sterilization by radiation exposure has a relative humidity of higher than 40%RH at 25°C, the amount of formed peroxide such as hydrogen oxide can be reduced as mentioned above. The present inventors have intensively researched a means capable of readily creating such a situation. As a result, it is found to be effective to surely realize a deoxidation system and to use a package which can stably envelope a blood purifier.

While sealing a blood purifier in a package after the radiation exposure is included in the scope of the present invention, it is preferable to carry out the radiation exposure on a blood purifier which is sealed in a package.

Preferable as a material for the package of the present invention is such a material that has a low oxygen permeability and/or a low water vapor permeability, specifically, at least has an oxygen permeability of 1 cm$^3$/(m$^2$.24hr.atm) or less (20°C and 90%RH), and a water vapor permeability of 5 g/(m$^2$.24hr.atm) or less (40°C and 90%RH). The replacement by the above described inert gas or the use of the above described oxygen scavenger is preferable to decrease the oxygen concentration. As the oxygen scavenger usable in the present invention, such an oxygen scavenger that has a deoxidating function and simultaneously has a moisture-releasing function is preferably used. Example of an agent which exhibit the deoxidation function include general-purpose oxygen scavengers such as sulfite, hydrogensulfite, dithionite, hydroquinone, catechol, resorcinol, pyrogallol, gallic acid, rongalite, ascorbic acid and/or a salt thereof, sorbose, glucose, lignin, dibutylhydroxytoluene, dibutylhydroxyanisole, metal powder (e.g. ferrous salt, iron powder, etc.) and the like. The oxygen scavenger is appropriately selected from these materials for use. The oxygen scavenger mainly containing metal powder, if needed, may contain, as an oxidation catalyst, one or more compounds selected from halogenated metal compounds such as sodium chloride, potassium chloride, magnesium chloride, calcium chloride, aluminum chloride, ferrous chloride, ferric chloride, sodium bromide, potassium bromide, magnesium bromide, calcium bromide, iron bromide, nickel bromide, sodium iodide, potassium iodide, magnesium iodide, calcium iodide, iron iodide, etc. As a method for imparting a moisture-releasing function to the hollow fiber membrane, a moisture-release type oxygen scavenger (e.g. Ageless(R) Z-200PT manufactured by Mitsubishi Gas Chemical Company, Inc.) or a porous carrier such as zeolite powder impregnated with moisture is included in a package together with the hollow fiber membrane. Additionally, other functional fillers such as a deodorant may be added. The form of the oxygen scavenger is not limited, and it may be in the form of powder, particles, mass or sheet; or it may be a sheet- or film-shaped oxygen scavenger obtained by dispersing the oxygen absorber composition in a thermoplastic resin. To optimize the humidity in the ambient atmosphere around the hollow fiber membrane, the deoxidation time or the storage temperature may be increased after the blood purifier and the moisture release type oxygen scavenger are sealed in the package satisfying the above conditions.

As a result of the above intensive researches, it is found that the amount of peroxides such as hydrogen peroxide can be effectively decreased by controlling the humidity of the ambient atmosphere around the hollow fiber membrane to higher than 40%RH, provided that the moisture content of the hollow fiber membrane is controlled to 0.2 to 7 mass %.

The peroxide, typically, hydrogen peroxide, gives not a little influence on the long-term storage stability of the blood purifier. The peroxide in the blood purifier attacks the polymers constituting the membrane by its chemical reaction together with free radicals, while expanding the chemical reaction with time. Needless to say, it is preferable that the amount of hydrogen peroxide extracted from the hollow fiber membrane should be small immediately after the sterilization by radiation exposure. Further, it is preferable that the amount of hydrogen peroxide extracted from the hollow fiber membrane should be 10 ppm or less after 3 months has passed since the sterilization of the blood purifier by radiation exposure. This is important to sufficiently ensure the stability of the blood purifier after the passage of a half year, one year and 3 years which is generally regarded as the guarantee period of the blood purifier. To meet this guarantee and the safety of the blood purifier, the amount of the peroxide extracted from the membrane is preferably 8 ppm or less, more preferably 5 ppm or less.

According to the present inventors' examination, it is experimentally confirmed that, when the amount of the hydrogen peroxide extracted from the hollow fiber membrane is 10 ppm after the passage of 3 months from the radiation exposure, the deterioration and disintegration of the hollow fiber membrane can be inhibited for at least 3 years thereafter (in other words, the amount of eluted hydrogen peroxide does not increase), in so far as the blood purifier is not taken out from the package.

An appropriate combined use of the preceding manufacturing methods which were intensively and extensively studied by the present inventors makes it possible to provide a blood purifier excellent in safety, biocompatibility and long-term storage stability.

EXAMPLES

Hereinafter, the effectiveness of the present invention will be explained by way of Examples thereof, which should not be construed as limiting the scope of the present invention in any way. The methods of evaluating the physical properties in the following Examples are described below.

1. Coefficient of Water Permeability

The circuit on the side of the blood outlet in a dialyzer (on the side of the outlet from a pressure-measuring point) is blocked with a forceps to make a full filtration circuit. A compression tank is charged with pure water maintained at 37°C, and the pure water is fed to the dialyzer insulated in a constant-temperature bath of 37°C while the pressure in the bath is being controlled with a regulator, and the mass of a filtrate flowing out of the side of the dialyzate is measured in the order of up to 1/100 g. The difference in pressure of the membranes (TMP) is expressed by the equation:

$$TMP = (Pi + Po)/2$$

[in the equation, Pi represents the pressure on the side of the inlet of the dialyzer; and Po, the pressure on the side of the outlet thereof]. The TMP is varied at four points, and the flow amount of the filtrate is measured, and the coefficient of water permeability (mL/hr./mmHg) is calculated from the slope indicting the relationship between TMP and the flow amount of the filtrate. At this point of time, the coefficient of correlation between TMP and the flow amount of the filtrate must be 0.999 or more. To reduce an error in pressure loss due to the circuit, TMP is measured within a pressure range of 100 mmHg or lower. The coefficient of water permeability of the hollow fiber membrane is calculated from the area of the membranes and the coefficient of water permeability of the dialyzer:

$$UFR(H) = UFR(D)/A$$

[in the equation, UFR(H) represents the coefficient of water permeability ($mL/m^2/hr/mmHg$) of the hollow fiber membrane; and UFR(D), the coefficient of water permeability (mL/hr/mmHg) of the dialyzer; and A, the area ($m^2$) of the membranes in the dialyzer].

2. Calculation of the Area of Membranes

The area of the membranes in the dialyzer is calculated based on the inner diameter of the hollow fiber membrane as a reference:

$$A = n \times \pi \times d \times L$$

[in the equation, n represents the number of the hollow fiber membranes in the dialyzer; $\pi$ represents the ratio of the circumference of a circle to its diameter; d represents the inner diameter (m) of the hollow fiber membrane; and L represents the effective length (m) of the hollow fiber membranes in the dialyser].

3. Burst Pressure

The dialyzate side of a module comprising about 10,000 hollow fiber membranes is filled with water and is then capped. A dried air or nitrogen is fed from the side of blood at a room temperature so as to compress the hollow fiber membranes at a rate of 0.5 MPa/min. The pressure is increased so that the hollow fiber membranes are bursted by the compressed air. The air pressure is measured, when bubbles occur in the liquid filling the module on the side of the dialyzate, because of the bursting of the membranes. This air pressure is defined as a burst pressure.

4. Non-Uniformity of Thickness

The sections of 100 hollow fibers are observed through a projector of a magnification of 200. One hollow fiber whose section has the largest difference in thickness is selected from 100 hollow fibers in one field of view, and the thickness of this hollow fiber is measured at its thickest portion and its thinnest portion.
The non-uniformity of thickness = the thickness of the thinnest portion/the thickness of the thickest portion
In this regard, the thickness of a membrane is perfectly uniform when the non-uniformity of thickness is one.

5. Amount of Eluted Hydrophilic Polymer

A method of measuring the amount of polyvinyl pyrrolidone as a hydrophilic polymer, eluted from a membrane is described.
Extraction is made on membranes according to the method regulated in the approved standards for manufacturing dialyzer type artificial kidney, and polyvinyl pyrrolidone in the extract is determined by a colorimetric method.
In detail, pure water (100 ml) is added to hollow fiber membranes (1 g), and extraction is made on the hollow fiber membranes at 70°C for one hour. To the resultant extract (2.5 ml), a 0.2 mol aqueous citric acid solution (1.25 ml) and a 0.006N aqueous iodine solution (0.5 ml) are added, and the mixture is sufficiently mixed and is left to stand alone at a room temperature for 10 minutes. After that, the absorbance of the mixture is measured at 470 nm. The determination is made using polyvinyl pyrrolidone as a sample, based on the analytical curve determined according to the above method. In case of a wet blood purifier, physiological saline is allowed to pass through the passage on the side of dialyzate in the module at a rate of 500 mL/min. for 5 minutes, and then is allowed to pass through the passage on the side of blood in the module at a rate of 200 mL/min. After that, physiological saline is allowed to pass through from the side of the blood to the side of the dialyzate, at a rate of 200 mL/min. for 3 minutes, while being filtered. Then, the membranes are freeze-dried. The resultant dried membranes are used for the above determination.

6. Content of Hydrophilic Polymer in Outer Surface of Membrane

The content of a hydrophilic polymer to a hydrophobic polymer is determined by the X-ray photoelectron spectroscopy (ESCA method). Analysis using a polysulfone type polymer as a hydrophobic polymer and polyvinyl pyrrolidone as a hydrophilic polymer is herein described.
One hollow fiber membrane is applied on a sample table to be analyzed by the X-ray photoelectron spectroscopy (ESCA method). The conditions for the analysis are as follows:

Apparatus: ULVAC-PHI ESCA5800
Excitation X-ray: MgKα ray
X-ray output: 14 kV, 25 mA
Escape angle of photoelectron: 45°
Analyzed diameter: 400 $\mu m \phi$
Pass energy: 29.35 eV
Resolution: 0.125 eV/step
Degree of vacuum: about $10^{-7}$ Pa or less
The content of PVP in the surface of the membrane is calculated from the found value of nitrogen (N) and the found value of sulfur (S), by the following equation:

```
<Membrane of PES (polyether sulfone) admixed with PVP>
        Content of PVP (Hpvp) [mass %]
        = 100 X (N X 111)/(N X 111 + S X 232)


<Membrane of PSf (polysulfone) admixed with PVP>
        Content of PVP (Hpvp) [mass %]
        = 100 X (N X 111)/(N X 111 + S X 442)
```

7. Content of Hydrophilic Polymer in Membrane

Measurement using PVP as a hydrophilic polymer is described. A sample is dried with a vacuum drier at 80°C for 48 hours, and 10 mg of the dried sample is analyzed with a CHN coder (Model MT-6, manufactured by YANAKO BUNSEKI KOGYOSHA). The content of PVP is calculated from the content of nitrogen by the following equation.

```
The content of PVP (mass %) = the content of nitrogen
(mass %) X 111/14
```

8. Ratio of hole areas of Outer Surface of Hollow Fiber Membrane

The outer surface of a hollow fiber membrane is observed with an electron microscope of a magnification of 10,000 and photographed (SEM photograph). The obtained image is processed with an image analysis processing soft to determine the ratio of hole areas of the outer surface of the hollow fiber membrane. For example, "Image Pro Plus" (Media Cybernetics, Inc.) is used as the image analysis processing soft for measurement. The fetched image is subjected to an emphasis/filter operation so as to discriminate the hole portions from the closed portions. After that, the number of the holes is counted. If polymer chains of the lower layer are seen in the interiors of the holes, such holes are combined and regarded as one hole. The total (B) of the area (A) within the measured range and the area of the holes within the measured range is calculated, and the ratio of hole areas (%) is calculated by the equation: the ratio of hole areas (%) = B/A X 100. This calculation is repeated with respect to 10 fields of view, and an average of the results is found. Scale-setting is carried out as the initiating operation, and the holes on the boundary around the measured range are not excluded from the counting.

9. Average Hole Area of Open area of Outer Surface of Hollow Fiber Membrane

Counting is made in the same manner as in the above operation, to calculate the area of each hole. The holes on the boundary around the measured range are excluded from the counting. This calculation is repeated with respect to 10 fields of view, and an average of all the hole areas is calculated.

10. Thickness of Hollow Fiber Membrane

The sections of hollow fiber membranes are projected with a projector of a magnification 200. The inner diameters (A) and the outer diameters (B) of the hollow fibers with maximum, minimum and medium sizes within each field of view are measured, and the thickness of each hollow fiber membrane is calculated by the following calculation, and an average of the thicknesses of 90 hollow fiber membranes within 30 fields of view is calculated.

$$\text{The thickness of the membrane} = (B - A)/2$$

11. Concentration of Endotoxin

A dialyzate containing endotoxin at a concentration of 200 EU/L is fed from the dialyzate inlet of a module at a rate of 500 ml/min. so as to filter the endotoxin-containing dialyzate from the outer side of a hollow fiber membrane to the inner side thereof at a filtering rate of 15 ml/min. for 2 hours. The filtered dialyzate thus obtained is reserved, and the concentration of endotoxin in the reserved dialyzate is measured. The concentration of endotoxin is analyzed with a limulus ESII test wako (manufactured by Wako Pure Chemical Industries, Ltd.) according to the method (gelation-reversing method) described in the manual attached thereto.

12. Blood Leak Test

Bovine blood of which the coagulation is inhibited by the addition of citric acid is fed to a blood purifier comprising a module primed with physiological saline, at a rate of 200 mL/min., and is filtered at a rate of 20 mL/min. The resulting filtrate is returned to the blood to make up a circulating system. After 60 minutes has passed, the filtrate of the blood purifier is collected, and the reddish tone of the filtrate due to the leakage of blood cell is visually observed. This blood leak test is conducted using 30 blood purifiers in each of Examples and Comparative Examples, and the number of modules from which blood is leaked is counted.

13. Sticking tendency of Hollow Fiber Membranes

About 10,000 hollow fibers are bundled, and the bundle thereof is set in a module casing of 30 to 35 mmφ. The module casing is sealed with a two-pack polyurethane resin to make up a module. The leak test is conducted on 30 standard modules for each size. After that, the number of the modules having defects in the sealing with the urethane resin is counted.

14. Measurement of Oxygen Concentration in Package

The measurement is conducted by gas chromatography, using a column filled with a molecular sieve (13X-S Mesh 60/80 manufactured by GL Science), an argon gas as a carrier gas, and a thermal conductive type detector. The analysis is made at a column temperature of 60°C. The inner gas of a package is collected by directly piercing the unopened package with the needle of a syringe.

15. Measurement of Moisture in Hollow Fiber Membrane

An absolute dry method is employed to measure the moistures of hollow fiber membranes. The weight of about 1 g of hollow fiber membranes as a sample is precisely measured in the order of four figures below a decimal point. After that, the hollow fiber membranes are absolutely dried at 105°C for 3 hours, and then are sufficiently cooled to a room temperature. After that, the absolutely dried hollow fiber membranes are precisely weighed in the order of four figures below the decimal point. The moisture is calculated by the following equation.

```
Moisture (mass %) =
((the weight of non-dried membrane - the weight of
 the absolutely dried membrane)/the weight of
 the absolutely dried membrane)
 X 100
```

16. Measurement of Relative Humidity

The relative humidity referred to in the present invention is determined by the following equation, using a water vapor partial pressure (p) at a room temperature (25°C) and a saturated water vapor pressure (P) at a room temperature (25°C): Relative Humidity (%RH) = p/P X 100. The sensor of a hygrothermo-meter (Ondotori(R) RH type manufactured by T & D) is inserted into a package, and the package is sealed to carry out a continuous measurement.

17. Elution Test of Circuit According to Approved standards for Manufacturing Dialyzer Type Artificial Kidney Device

During a measurement, physiological saline (manufactured by Otsuka Pharmaceutical Co., Ltd.) as an initial washing liquid is allowed to flow on the blood side of a hollow fiber membrane type blood purifier at a flow rate of 100 ml/min. to perfectly fill the blood purifier, and the washing liquid (25 mL) is sampled for 15 seconds after the filling of the blood purifier. To confirm the amount of the eluted component after 5 minutes has passed since the start of washing, the washing liquid (25 mL) was sampled for 15 seconds after 5 minutes has passed since the start of washing. Ten milliliter is taken out af each of the samples, and this fraction of each sample is admixed with an aqueous solution of potassium permanganate (2.0 X 10-3 mol/1) (20 mL) and diluted hydrochloric acid (1 mL), and the mixture is boiled for 3 minutes. The mixture is cooled to a room temperature, and is admixed with an aqueous potassium iodide solution (1 mL). The mixture is sufficiently stirred and is left to stand for 10 minutes, followed by titration using an aqueous sodium thiosulfate solution (1.0 X 10-2 mol/1) . Separately, the same operation as conducted on the samples, is conducted on water which does not pass through the blood purifier. The difference between the amount of the aqueous sodium thiosulfate solution used for the titration of the water which does not pass through the blood purifier and the amount of the aqueous sodium thiosulfate solution used for the titration of each sample is defined as the amount of the aqueous solution of potassium permanganate consumed by the eluted component (or the consumed amount of the aqueous solution of potassium permanganate).

18. Amount of Eluted Hydrogen Peroxide

An extract (2.6 mL) obtained by the method described in the part of the UV (220-350 nm) absorbance-measuring method regulated in the approved standards for manufacturing dialyzer type artificial kidney devices is admixed with an ammonium chloride buffer (pH 8. 6) (0.2 mL) and a mixture of a hydrogen chloride solution of $TiCl_4$ and an aqueous solution of a Na salt of 4-(2-pyridylazo)resorcinol (equivalent in a molar ratio). The resulting mixture is admixed with a coloring reagent adjusted to 0.4 mM (0.2 mL). The mixture is heated at 50°C for 5 minutes, and then is cooled to a room temperature. The absorbance of the resultant solution is measured at 508 nm, and is determined from an analytic curve which is obtained by the similar measurement using a sample.

19. Measurement of Oxygen Permeability of Material for Package

An oxygen permeability of a material for a package is measured with an oxygen permeability measuring apparatus (OX-TORAN 100 manufactured by Modern Controls) under conditions of 20°C and 90%RH.

20. Measurement of Water vapor Permeability of Material for Package

A water vapor permeability of a material for a package is measured with a water vapor permeability measuring apparatus (PARMATRAN-W manufactured by Modern Controls) under conditions of 40°C and 90%RH.

(Example 1)

Polyether sulfone (SUMIKAEXCEL(R) 5200P, manufactured by Sumika Chem Tex Co., Ltd.) (17 mass %), polyvinyl pyrrolidone (COLIDONE(R)K-90 manufactured by BASF) (2.5 mass %), dimethylacetamide (DMAc) (77. mass %) and RO water (3 mass %) were homogeneously dissolved at 50°C, and then, the system was vacuumed up to -500 mmHg with a vacuum pump. After that, the system was immediately sealed so as not to change the composition of the membrane-forming solution due to the evaporation of the solvent or the like, and the system in this state was left to stand alone for 15 minutes. This operation was repeated three times so as to degas the membrane-forming solution. This solution was allowed to pass through sintered filters with hole sizes of each 15 $\mu$m in two stages, and then was extruded through a tube-in-orifice nozzle heated to 80°C, together with an aqueous solution of DMAc (60 mass %) as a void-forming agent which had been previously degassed for 30 minutes under a pressure of -700 mmHg. Then, the extruded semi-solid hollow fiber membrane was allowed to pass through a drying section with a length of 400 mm, which was blocked from an outer air by a spinning tube, and then was solidified in an aqueous solution of DMAc (20 mass %) heated to 60°C. The resultant hollow fiber membrane in a wet state was directly wound onto a hank. The slit of the tube-in-orifice nozzle used had an average width of 60 $\mu$m, a maximum width of 61$\mu$m and a minimum width of 59 $\mu$m, and the ratio of the maximum value to the minimum value of the width of the slit was 1.03. The draft ratio of the membrane-forming solution was 1.06. The absolute humidity of the drying section was 0.18 kg/kg in a dry air. The rollers used, with which the hollow fiber membranes came into contact during the spinning step, were planished at their surfaces, and all the guides used were matte-finished at their surfaces.

The bundle of 10,000 hollow fiber membranes as obtained above was wrapped in a polyethylene film which was matte-finished at its surface on the side of the bundle, and then was washed in hot water of 80°C for 30 minutes. This washing was repeated 4 times. After the completion of washing, the bundle of the membranes was dried under a nitrogen atmosphere of 40°C. The inner diameter of the resultant hollow fiber membrane was 198.5 $\mu$m, and the thickness of the membrane was 28.5 $\mu$m. The content of the hydrophilic polymer in the hollow fiber membrane was measured. As a result, the content thereof was 4.3 mass %.

A blood purifier was assembled using the hollow fiber membranes thus obtained, and was subjected to a leak test. As a result, no failure in adhesion, attributed to the sticking of the hollow fiber membranes, was observed.

The blood purifier was filled with RO water previously degassed, and was exposed to $\gamma$-ray to 25 kGy for crosslinking the hydrophilic polymer. After the $\gamma$-ray exposure, the hollow fiber membranes were cut out from the blood purifier, and the cut pieces of the hollow fiber membranes were subjected to an elution test. As a result, the amount of the eluted PVP was 8 ppm, which was a level of no problem.

The blood purifiers were charged with a compressed air under a pressure of 0.1 MPa, and several of the blood purifiers which showed decrease of 30 mmAq or less in pressure in 10 seconds were regarded as accepted products to the leak tests, and such accepted blood purifiers were used in the following test. The outer surface of the hollow fiber membrane removed from the blood purifier was observed with a microscope. As a result, no defect such as flaws or the like was observed. A fresh bovine blood admixed with citric acid was allowed to pass through the blood purifier at a flow rate of 200 mL/min. and at a filtering rate of 10 mL/min. As a result, no leakage of blood cell was observed. The amount of the endotoxin filtered from the outside of the hollow fiber membrane to the inside thereof was smaller than the limit for detection, which was a level of no problem.

The blood purifier and Ageless(R) Z-200PT (manufactured by Mitsubishi Gas Chemical Company, Inc.) were put in a package having an oxygen permeability of 0.5 cm$^3$/ (m$^2$.24hr.atm) (20°C, 90%RH) and a water vapor permeability of 3 g/(m$^2$.24hr.atm) (40°C, 90%RH), and the inner atmosphere of the package was replaced with a nitrogen gas. After that, the package was sealed and was left to stand at a room temperature for 12 hours, and was then exposed to $\gamma$-ray to 25 kGy for sterilizing the blood purifier. The moisture content of the hollow fiber membrane to its weight was 2.5 mass %; the relative humidity around the hollow fiber membrane was 60%RH (25°C); and the oxygen concentration in the package was 0.05%. As a result of the evaluation of the resultant blood purifier, the amount of the eluted component from the hollow fiber membrane was 7 ppm; the amount of the consumed aqueous potassium permanganate solution was 2 mL per 1 m$^2$ of the inner surface of the hollow fiber membrane; and the amount of the eluted hydrogen peroxide from the hollow fiber membrane found after the storage of the blood purifier under an atmosphere of 25°C and 50%RH for 3 months was 3 ppm. The results of other analyses are shown in Table 1.

(Comparative Example 1)

A bundle of wet hollow fiber membranes was obtained in the same manner as in Example 1, except that the same membrane-forming solution as that of Example 1 was not allowed to pass through a filter, and that the resultant membranes were not washed. The hollow fiber membranes thus obtained were used to assemble a blood purifier. The blood purifier was filled with RO water, and exposed to γ-ray to an absorbed dose of 25 kGy so as to crosslink the hydrophilic polymer. The inner diameter of the resultant hollow fiber membrane was 199 μm, and the thickness thereof was 28 μm. The content of the hydrophilic polymer in the hollow fiber membrane was measured, and it was 9.6 mass %. The hollow fiber membranes were removed from the blood purifier after the exposure to γ-ray, and observed with a microscope. As a result, some of the membranes had knob-like defects which seemed to occur due to the inclusion of the non-dissolved components. The blood purifiers were charged with a compressed air under a pressure of 0.1 MPa, and modules which showed decrease of 30 mmAq or less in pressure in 10 seconds were used in tests. In the blood leak tests using bovine blood, the blood leaked from 3 modules out of 30 modules. This was because the thin portions of such modules had insufficient strength and/or some defects due to the non-uniformity in thickness and the low burst pressures. As a result of the endotoxin-permeating tests, endotoxin having permeated to the inner side of the hollow fiber membrane was observed. This was because, since no washing was done, the content of PVP in the outer surface of the hollow fiber membrane increased, which made it easy for the endotoxin to pass through the membrane.

The blood purifier and Ageless(R) Z-200PT (manufactured by Mitsubishi Gas Chemical Company, Inc.) were put in a package having an oxygen permeability of 0.5 cm$^3$/ (m$^2$.24hr.atm) (20°C, 90%RH) and a water vapor permeability of 3 g/(m$^2$.24hr.atm) (40°C, 90%RH), and the inner atmosphere of the package was replaced with a nitrogen gas. After that, the package was sealed and was left to stand at a room temperature for 12 hours, and was then exposed to γ-ray to 25 kGy for sterilizing the blood purifier. The moisture content of the hollow fiber membrane to its weight was 2.8 mass %; the relative humidity around the hollow fiber membrane was 70%RH (25°C); and the oxygen concentration in the package was 0.08%. As a result of the evaluation of the resultant blood purifier, the amount of the eluted component from the hollow fiber membrane was 8 ppm; the amount of the consumed aqueous potassium permanganate solution was 3 mL per 1 m$^2$ of the inner surface of the hollow fiber membrane; and the amount of the eluted hydrogen peroxide from the hollow fiber membrane found after the storage of the blood purifier under an atmosphere of 25°C and 50%RH for 3 months was 3 ppm. The results of other analyses are shown in Table 1.

(Comparative Example 2)

Polyethersulfone (SUMICAEXEL(R)5200P, manufactured by SUMIKA CHEM TEX) (16 mass %), polyvinyl pyrrolidone (KOLIDONE(R)K-90, manufactured by BASF) (6 mass %), DMAc (75 mass %) and water (3 mass %) were dissolved at 50°C. The interior space of the system was decompressed up to -500 mmHg with a vacuum pump, and then was immediately sealed so as not to change the composition of the membrane-forming solution due to the evaporation of the solvent or the like. Then, the system was left to stand alone for 15 minutes. This operation was repeated three times to degas the membrane-forming solution. This solution was allowed to pass through a filter with a hole size of 100 μm, and then was extruded together with an aqueous solution of DMAc (30 mass %) as a void-forming agent which had been previously degassed under a pressure of -700 mmHg for 2 hours, through a tube-in-orifice nozzle heated to 60°C. The resultant semi-solid hollow fiber membrane was allowed to pass through a drying section with a length of 600 mm shielded by a spinning tube from an external and then was solidified in an aqueous solution of DMAc (concentration: 10 mass %) of 60°C. The slit of the tube-in-orifice nozzle used had an average width of 100 μm, a maximum width of 110 μm and a minimum width of 90 μm; the ratio of the maximum value to the minimum value of the width of the slit was 1.22; and the draft ratio was 2.41. The absolute humidity of the drying section was 0.12 kg/kg in a dry air. The resultant hollow fiber membrane was allowed to pass through a water bath of 40°C for 45 seconds so as to remove the solvent and an excess of the hydrophilic polymer, and then was directly wound up in a wet state, and dried in an air at 50°C. The inner diameter of the resultant hollow fiber membrane was 197.8 μm, and the thickness of the membrane was 29.2 μm. The content of the hydrophilic polymer in the hollow fiber membrane was 7.4 mass %.

A blood purifier was assembled using the hollow fiber membranes thus obtained. The blood purifier was filled with pure water and exposed to γ-ray to an absorbed dose of 25 kGy for crosslinking the hydrophilic polymer. The hollow fiber membranes were cut out from the blood purifier after the exposure to γ-ray and subjected to an elution test. As a result, the amount of the eluted PVP was 12 ppm. This was because of the insufficient washing of the hollow fiber membranes. The blood purifiers were charged with a compressed air under a pressure of 0.1 MPa to select modules which showed decrease of 30 mmAq or less in pressure in 10 seconds, and such modules were used in tests. Blood cell was leaked from 2 modules out of 30 modules in the blood leak tests using bovine blood. It was considered that this was attributed to the occurrence of pin holes and/or breakage of the membranes because of the small non-uniformity and too large sizes of the holes of the outer surfaces of the membranes. As a result of the endotoxin-permeating tests, endotoxin filtered from the outside of the hollow fibers to the inside thereof was detected. It was supposed that the larger amount of PVP in the outer surface of the membrane and the higher ratio of hole areas of the membrane facilitated the permeation of endotoxin. Subsequent sterilization was not done, since the amount of the eluted PVP found before the sterilization by radiation exposure was large. The results of other analyses are shown in Table 1.

(Example 2)

Polyether sulfone (SUMIKAEXCEL(.R)4800P, manufactured by Sumika Chem Tex Co., Ltd.) (18 mass %), polyvinyl pyrrolidone (COLIDONE(R)K-90 manufactured by BASF) (3.5 mass %), dimethylacetoamide (DMAc) (73.5 mass %) and water (5 mass %) were dissolved at 50°C. Then, the system was vacuumed up to -700 mmHg with a vacuum pump. After that, the system was immediately sealed so as not to change the composition of the membrane-forming solution due to the evaporation of the solvent or the like, and the system was left to stand alone for 10 minutes. This operation was repeated three times to degas the membrane-forming solution. This solution was allowed to pass through filters with hole sizes of each 15 $\mu$m in two stages, and then was extruded through a tube-in-orifice nozzle heated to 70°C, together with an aqueous solution of DMAc (50 mass %) as a void-forming agent, which had been previously degassed for 2 hours under a pressure of -700 mmHg. Then, the semi-solid hollow fiber membrane was allowed to pass through an air gap with a length of 300 mm, which was blocked from an external air by a spinning tube, and then was solidified in water of 60°C. The slit of the tube-in-orifice nozzle used had an average width of 45 $\mu$m, a maximum width of 45.5 $\mu$m and a minimum width of 44.5 $\mu$m, and the ratio of the maximum value to the minimum value of the width of the slit was 1.02. The draft ratio was 1.06. The absolute humidity of the drying section was 0.12 kg/kg in a dry air. The hollow fiber membrane removed from the solidifying bath was allowed to pass through a water washing bath of 85°C for 45 seconds to remove the solvent and an excess of the hydrophilic polymer. After that, the resultant membrane was wound onto a hank. The rollers used to change the fiber path in the spinning step were planished at their surfaces, and the stationary guides were matte-finished at their surfaces.

The bundle of about 10,000 hollow fiber membranes as obtained above was wrapped in the same polyethylene film as that used in Example 1, and then was dipped and washed in an aqueous solution of isopropanol (40 vol. %) of 30°C for 30 minutes. This operation was repeated twice, and then, the bundle of the membranes was washed with water with which the aqueous isopropanol solution was replaced. After the completion of washing, the bundle of the membranes was dried under a nitrogen stream of 60°C. The inner diameter of the resultant membrane was 198 $\mu$m, and the thickness thereof was 29 $\mu$m. The content of the hydrophilic polymer in the hollow fiber membrane was measured. As a result, the content thereof was 7.3 mass %. A blood purifier was assembled using the hollow fiber membranes thus obtained, and was used for a leak test. As a result, no failure in adhesion, attributed to the sticking of the hollow fiber membranes, was observed. The blood purifier was used in the following analysis, without crosslinking the hydrophilic polymer. The hollow fiber membranes were cut out from the blood purifier which had not been exposed to $\gamma$-ray, and were subjected to an elution test. As a result, the amount of the eluted PVP was 6 ppm, which was evaluated as good. Further, the hollow fiber membranes were removed from the blood purifier, and the outer surfaces thereof were observed with a microscope. As a result, no defect such as flaws or the like was observed. In the blood leak test using bovine blood, any of the membranes showed no leakage of blood cell. As a result of the endotoxin-permeating test, the amount of the endotoxin filtered from the outside of the hollow fibers to the inside thereof was smaller than the limit for detection, which was within the level of no problem.

The blood purifier and Ageless(R) Z-20OPT (manufactured by Mitsubishi Gas Chemical Company, Inc.) were put in a package having an oxygen permeability of 0.5 cm$^3$/(m$^2$.24hr.atm) (20°C, 90%RH) and a water vapor permeability of 3 g/(m$^2$.24hr.atm) (40°C, 90%RH), and the inner atmosphere of the package was replaced with a nitrogen gas. After that, the package was sealed and was left to stand at a room temperature for 36 hours, and was then exposed to $\gamma$-ray to 25 kGy for sterilizing the blood purifier. The moisture content of the hollow fiber membrane to its weight was 2.2 mass %; the relative humidity around the hollow fiber membrane was 60%RH (25°C); and the oxygen concentration in the package was 0.03%. As a result of the evaluation of the resultant blood purifier, the amount of the eluted component from the hollow fiber membrane was 8 ppm; the amount of the consumed aqueous potassium permanganate solution was 2 mL per 1 m$^2$ of the inner surface of the hollow fiber membrane; and the amount of the eluted hydrogen peroxide from the hollow fiber membrane found after the storage of the blood purifier under an atmosphere of 25°C and 50%RH for 3 months was 2 ppm. The results of other analyses are shown in Table 1.

(Comparative Example 3)

Polyethersulfone (SUMICAEXEL(R)7800P, manufactured by Sukika Chem Tex Co., Ltd.) (22 mass %), polyvinyl pyrrolidone (KOLIDONE(R)K-30, manufactured by BASF) (9 mass %), DMAc (66 mass %) and water (3 mass %) were dissolved at 50°C. The interior of the system was decompressed up to -350 mmHg with a vacuum pump, and then was immediately sealed so as not to change the composition of the membrane-forming solution due to the evaporation of the solvent or the like, and then was left to stand alone for 30 minutes. This operation was repeated twice to degas the membrane-forming solution. The resultant solution was allowed to pass through filters with hole sizes of each 30 $\mu$m in two stages, and then was extruded through a tube-in-orifice nozzle heated to 50°C, together with an aqueous solution of DMAc (50 mass %) as a void-forming agent which had been previously degassed under reduced pressure. The resultant semi-solid hollow fiber membrane was allowed to pass through an air gap with a length of 300 mm shielded by a spinning tube from an external air and then was solidified in water of 50°C. The slit of the tube-in-orifice nozzle used had an average width of 45 $\mu$m, a maximum width of 45.5 $\mu$m and a minimum width of 44.5 $\mu$m; the ratio of the maximum value to the minimum value of the width of the slit was 1.02; and the draft ratio was 1.06. The absolute humidity of the drying section was 0.07 kg/kg in a dry air. The resultant hollow fiber membrane removed from the above bath was allowed to pass through a water bath of 40°C for 45 seconds so as to remove the solvent and an excess of the hydrophilic polymer, and then was wound up. The bundle of 10,000 hollow fiber membranes thus obtained were directly dried in an air at 40°C without washing. The inner diameter of the resultant hollow fiber membrane was 199.5 $\mu$m, and the thickness thereof was 29.0 $\mu$m. The content of the hydrophilic polymer in the hollow fiber membrane was 7.7 mass %.

After the drying, some of the hollow fiber membranes in the bundle were stuck to one another. Thus, an adhesive resin was not successfully inserted between each of the hollow fiber membranes at their end portions, when a blood purifier was assembled using such hollow fiber membranes. Therefore, it was impossible to assemble the blood purifier. The results of the analysis are shown in Table 1.

(Comparative Example 4)

The same membrane-forming solution as that used in Example 1 was allowed to pass through filters with hole sizes of 30$\mu$m and 15 $\mu$m, respectively, in two stages, and was then extruded through a tube-in-orifice nozzle heated to 80°C, together with an aqueous solution of DMAc (60 mass %) as a void-forming agent which had been previously degassed under reduced pressure. The resultant semi-solid hollow fiber membrane was allowed to pass through a drying section with a length of 400 mm shielded by a spinning tube from an external air and then was solidified in a bath holding RO water of 70°C. The slit of the tube-in-orifice nozzle used had an average width of 60 $\mu$m, a maximum width of 62 $\mu$m and a minimum width of 58 $\mu$m; the ratio of the maximum value to the minimum value of the width of the slit was 1.07; and the draft ratio was 1.06. The absolute humidity of the drying section was 0.23 kg/kg in a dry air. The resultant hollow fiber membrane removed from the above bath was dipped in a water bath of 60°C for 45 seconds, and then was wound up and dried in a dry oven of 70°C. The inner diameter of the resultant hollow fiber membrane was 200 $\mu$m, and the thickness thereof was 31 $\mu$m. The content of the hydrophilic polymer in the hollow fiber membrane was 6.3 mass %.

A blood purifier was assembled using the hollow fiber membranes thus obtained, and was subjected to an air leak test. As a result, bubbles occurred from the adhered portion of the module. It was considered that the failure in adhesion was attributed to the sticking of the hollow fibers. The hollow fiber membranes were cut out from the blood purifier which had not undergone a crosslinking treatment, and were subjected to an elution test. As a result, the amount of the eluted PVP was 12 ppm. It was considered that this result was attributed to the insufficient washing of the hollow fiber membranes and the non-crosslinked hydrophilic polymer. Such blood purifiers were charged with a compressed air under a pressure of 0.1 MPa to select modules which showed decrease of 30 mmAq or less in pressure in 10 seconds, and such modules were used in tests. As a result of the blood leak test using bovine blood, no leakage of blood cell was observed. As a result of the endotoxin-permeating test, the concentration of endotoxin in the filtrate was 10 EU/L, which was a slightly high level. Subsequent sterilization was not done, since the amount of the eluted PVP found before the sterilization by radiation exposure was large, and since there was a problem of the permeation of endotoxin. The results of the analysis of the blood purifier are shown in Table 1.

(Comparative Example 5)

Polyethersulfone (SUMIKAEXEL (R) 5200P, manufactured by Sumika Chem Tex., Co ., Ltd.) (17 mass %), polyvinyl pyrrolidone (KOLIDONE(R)K-90, manufactured by BASF) (7.5 mass %), DMAc (72.5 mass %) and water (3 mass %) were dissolved at 50°C. The interior of the system was decompressed up to -500 mmHg with a vacuum pump, and then was immediately sealed so as not to change the composition of the membrane-forming solution due to the evaporation of the solvent or the like, and then was left to stand alone for 30 minutes. This operation was repeated three times to degas the membrane-forming solution. The resultant membrane-forming solution was extruded through a tube-in-orifice nozzle heated to 50°C, without filtering, together with an aqueous solution of DMAc (75 mass %) as a void-forming agent which had been previously degassed under reduced pressure. The resultant semi-solid hollow fiber membrane was allowed to pass through an air gap with a length of 600 mm, shielded by a spinning tube from an external air, and then was solidified in water of 70°C. The slit of the tube-in-orifice nozzle used had an average width of 60 $\mu$m, a maximum width of 64 $\mu$m and a minimum width of 56 $\mu$m; the ratio of the maximum value to the minimum value of the width of the slit was 1.14; and the draft ratio was 1.06. The absolute humidity of the drying section was 0.17 kg/kg in a dry air. The resultant hollow fiber membrane was washed with water to remove the solvent, and then was wound up to make up a bundle of about 10,000 hollow fiber membranes. Then, the bundle of the membranes was dipped in an aqueous solution of glyceline (30 mass %) of 50°C for one hour and dried at 80°C. The inner diameter of the resultant hollow fiber membrane was 197 μm, and the thickness thereof was 30 μm. The content of the hydrophilic polymer in the hollow fiber membrane was 6.1 mass %.

The bundle of the hollow fiber membranes thus obtained showed no sticking of the hollow fibers, since the surfaces of the membranes were coated with glyceline. However, a blood purifier assembled using this bundle of the hollow fiber membranes could not ensure sufficient safety, because of the large amount of the urethane oligomer at the end portions of the hollow fibers. The blood purifier filled with water was exposed to γ-ray to an absorbed dose of 25 kGy. The hollow fiber membranes were cut out from the blood purifier after the exposure to γ-ray, and were subjected to an elution test. As a result, the amount of the eluted PVP was 13 ppm. It was considered that this result was attributed to the insufficient washing of the hollow fiber membranes and the influence of the glyceline in the filler liquid which hindered the crosslinking of the hydrophilic polymer. Such blood purifiers were charged with a compressed air under a pressure of 0.1 MPa to select modules which showed decrease of 30 mmAq or less in pressure in 10 seconds, and such modules were used in tests. As a result of the blood leak tests using bovine blood, the leakage of blood cell was observed in 4 modules out of 30 modules. It was considered that this result was attributed to the small non-uniformity in thickness and the too large sizes of holes of the outer surfaces of the membranes. As a result of the endotoxin-permeating test, the concentration of endotoxin filtered from the outside of the hollow fibers to the inside thereof had a very high level. It was considered that this result was attributed to the large ratio of hole areas and the large hole area of the outer surfaces of the membranes. Subsequent sterilization was not done, since the amount of the eluted PVP found before the sterilization by radiation exposure was large, and since the amount of the permeated endotoxin was large. The results of other analyses are shown in Table 1.

(Example 3)

Polysulfone (P-3500, manufactured by AMOKO) (18 mass %), polyvinyl pyrrolidone (K-60 manufactured by BASF) (9 mass %), DMAc (68 mass %) and water (5 mass %) were dissolved at 50°C, and then, the system was vacuumed up to -300 mmHg with a vacuum pump. After that, the system was immediately sealed so as not to change the composition of the membrane-forming solution due to the evaporation of the solvent or the like, and the system was left to stand alone for 15 minutes. This operation was repeated three times to degas the membrane-forming solution. This solution was allowed to pass through filters with hole sizes of each 15μ m in two stages, and then was extruded through a tube-in-orifice nozzle heated to 40°C, together with an aqueous solution of DMAc (35 mass %) as a void-forming agent which had been previously degassed under reduced pressure. Then, the resultant semi-solid hollow fiber membrane was allowed to pass through an air gap with a length of 600 mm, which was blocked from an external air by a spinning tube, and then was solidified in water of 50°C. The slit of the tube-in-orifice nozzle used had an average width of 60 μm, a maximum width of 61 μm and a minimum width of 59 μm. The ratio of the maximum value to the minimum value of the width of the slit was 1.03, and the draft ratio was 1.01. The absolute humidity of the drying section was 0.07 kg/kg in a dry air. The hollow fiber membrane removed from the solidifying bath was allowed to pass through a water washing bath of 85°C for 45 seconds to remove the solvent and an excess of the hydrophilic polymer. After that, the resultant membrane was wound up to make up a bundle of about 10,000 hollow fiber membranes. The bundle of the hollow fiber membranes was dipped in pure water and washed in an autoclave at 121°C for one hour. After the washing, the bundle of the membranes was wrapped in the same polyethylene film as that used in Example 1, and dried under a nitrogen stream of 45°C. The rollers used to change the fiber path in the spinning step were planished at their surfaces, and the stationary guides were matte-finished at their surfaces. The inner diameter of the resultant membrane was 201 μm, and the thickness thereof was 43 μm. The content of the hydrophilic polymer in the hollow fiber membrane was measured. As a result, the content thereof was 8.8 mass %.

A module was made up of the resultant hollow fiber membranes, and was used for a leak test. As a result, no failure in adhesion, attributed to the sticking of the hollow fibers, was observed. A blood purifier was assembled using the hollow fiber membranes thus obtained. The blood purifier was filled with RO water, and then was exposed to γ-ray to an absorbed dose of 25kGy to crosslink the hydrophilic polymer. The hollow fiber membranes were cut out from the blood purifier after the exposure to γ-ray, and were subjected to an elution test. As a result, the amount of the eluted PVP was 7 ppm, which was in the level of no problem. Such blood purifiers were charged with compressed air under a pressure of 0.1 MPa to select modules which showed decrease of 30 mmAq or less in pressure in 10 seconds, as accepted products to the leak tests, and such modules were used in the following tests. Further, the hollow fiber membranes were removed from the blood purifier, and the outer surfaces thereof were observed with a microscope. As a result, no defect such as flaws or the like was observed. Fresh bovine blood admixed with citric acid was allowed to pass through the blood purifier at a flow rate of 200 mL/min. and at a filtering rate of 10 mL/min. As a result, no leakage of blood cell was observed. The amount of the endotoxin filtered from the outside of the hollow fibers to the inside thereof was smaller than the limit for detection, which was within the level of no problem.

The blood purifier and Ageless(R) Z-200PT (manufactured by Mitsubishi Gas Chemical Company, Inc.) were put in a package having an oxygen permeability of 0.5 cm$^3$/(m$^2$.24hr.atm) (20°C, 90%PH) and a water vapor permeability of 3 g/ (m$^2$.24hr.atm) (40°C, 90%RH), and the inner atmosphere of the package was replaced with a nitrogen gas. After that, the package was sealed and was left to stand at a room temperature for 36 hours, and was then exposed to γ-ray to 25 kGy for sterilizing the blood purifier. The moisture content of the hollow fiber membrane to its weight was 3.1 mass %; the relative humidity around the hollow fiber membrane was 70%RH (25°C); and the oxygen concentration in the package was 0.05%. As a result of the evaluation of the resultant blood purifier, the amount of the eluted component from the hollow fiber membrane was 6 ppm; the amount of the consumed aqueous potassium permanganate solution was 2 mL per 1 m$^2$ of the inner surface of the hollow fiber membrane; and the amount of the eluted hydrogen peroxide found after the storage of the blood purifier under an atmosphere of 25°C and 50%RH for 3 months was 4 ppm. The results of other analyses are shown in Table 1.

(Example 4)

Polysulfone (P-1700, manufactured by AMOKO) (17 mass %), polyvinyl pyrrolidone (K-60 manufactured by BASF) (5 mass %), DMAc (68 mass %) and water (5 mass %) were dissolved at 50°C, and then, the system was vacuumed up to -400 mmHg with a vacuum pump. After that, the system was immediately sealed so as not to change the composition of the membrane-forming solution due to the evaporation of the solvent or the like, and the system was left to stand alone for 30 minutes. This operation was repeated three times to degas the membrane-forming solution. This solution was allowed to pass through filters with hole sizes of each 15μ m in three stages, and then was extruded through a tube-in-orifice nozzle heated to 40°C, together with an aqueous solution of DMAc (35 mass %) as a void-forming agent which had been previously degassed under reduced pressure. Then, the resultant semi-solid hollow fiber membrane was allowed to pass through an air gap with a length of 600 mm, which was blocked from an external air by a spinning tube, and then was solidified in water of 50°C. The slit of the tube-in-orifice nozzle used had an average width of 60 μm, a maximum width of 61 μm and a minimum width of 59 μm. The ratio of the maximum value to the minimum value of the width of the slit was 1.03, and the draft ratio was 1.01. The absolute humidity of the drying section was 0.12 kg/kg in a dry air. The hollow fiber membrane removed from the solidifying bath was allowed to pass through a water washing bath of 85°C for 45 seconds to remove the solvent and an excess of the hydrophilic polymer, After that, the resultant membrane was wound up to make up a bundle of about 10,000 hollow fiber membranes. The bundle of the hollow fiber membranes was dipped in pure water and washed in an autoclave at 121°C for one hour. After the washing, the bundle of the membranes was wrapped in a polyethylene film, and dried under a nitrogen stream of 45°C. The rollers used to change the fiber path in the spinning step were planished at their surfaces, and the stationary guides were matte-finished at their surfaces. The inner diameter of the resultant hollow fiber membrane was 201 μm, and the thickness thereof was 46 μm. The content of the hydrophilic polymer in the hollow fiber membrane was measured. As a result, the content thereof was 5.2 mass %.

A module for use in evaluation was assembled using the resultant hollow fiber membranes, and was used in a leak test. As a result, no failure in adhesion, attributed to the sticking of the hollow fibers, was observed. A blood purifier was assembled using the hollow fiber membranes thus obtained. The blood purifier was filled with RO water, and exposed to γ-ray to an absorbed dose of 25kGy to crosslink the hydrophilic polymer. The hollow fiber membranes were cut out from the blood purifier after the exposure to γ-ray, and were subjected to an elution test. As a result, the amount of the eluted PVP was 7 ppm, which was in the level of no problem. Such blood purifiers were charged with a compressed air under a pressure of 0.1 MPa to select modules which showed decrease of 30 mmAq or less in pressure in 10 seconds, as accepted products to the leak tests, and such modules were used in the following tests. Further, the hollow fiber membranes were removed from the blood purifier, and the outer surfaces thereof were observed with a microscope. As a result, no defect such as flaws or the like was observed. Fresh bovine blood admixed with citric acid was allowed to pass through the blood purifier at a flow rate of 200 mL/min. and at a filtering rate of 10 mL/min. As a result, no leakage of blood cell was observed. The amount of the endotoxin filtered from the outside of the hollow fibers to the inside thereof was smaller than the limit or detection, which was within the level of no problem.

The blood purifier and Ageless(R) Z-200PT (manufactured by Mitsubishi Gas Chemical Company, Inc.) were put in a package having an oxygen permeability of 0.5 cm$^3$/ (m$^2$.24hr.atm) (20°C, 90%RH) and a water vapor permeability of 3 g/(m$^2$.24hr.atm) (40°C, 90%RH), and the inner atmosphere of the package was replaced with a nitrogen gas. After that, the package was sealed and was left to stand at a room temperature for 36 hours, and was then exposed to γ-ray to 25 kGy for sterilizing the blood purifier. The moisture content of the hollow fiber membrane to its weight was 2.9 mass %; the relative humidity around the hollow fiber membrane was 70%RH (25°C); and the oxygen concentration in the package was 0.05%. As a result of the evaluation of the resultant blood purifier, the amount of the eluted component from the hollow fiber membrane was 4 ppm; the amount of the consumed aqueous potassium permanganate solution was 1 mL per 1 m$^2$ of the inner surface of the hollow fiber membrane; and the amount of the eluted hydrogen peroxide found after the storage of the blood purifier under an atmosphere of 25°C and 50%RH for 3 months was 6 ppm. The results of other analyses are shown in Table 1.

(Example 5)

Hollow fiber membranes were obtained in the same manner as in Example 1. A blood purifier was made up of these hollow fiber membranes and was then subjected to a leak test. As a result, no failure in adhesion attributed to the sticking of the hollow fiber membranes was observed. The blood purifier was subjected to a subsequent analysis without crosslinking the hydrophilic polymer. The hollow fiber membranes were cut out of the blood purifier which had not been exposed to γ-ray, and subjected to an elution test. As a result, the amount of the eluted PVP was 6 ppm which was a sufficient level. The hollow fiber membranes were removed from the blood purifier, and the outer surfaces of the hollow fiber membranes were observed with a microscope. As a result, no defect such as flaws or the like was observed. In a blood leak test using bovine blood, no leakage of blood cells was observed. In an endotoxin permeation test, the amount of endotoxin filtered from the outside of the hollow fiber membranes to the inside thereof was smaller than the limit of detection, which was in the level of no problem.

The blood purifier was put in a package having an oxygen permeability of $0.5\,cm^3/(m^2.24hr.atm)$ (20°C, 90%RH) and a water vapor permeability of $3\,g/(m^2.24hr.atm)$ (40°C, 90%RH), and the inner atmosphere of the package was replaced with a nitrogen gas. After that, the package was sealed and was then exposed to γ-ray to 25 kGy for sterilizing the blood purifier. The moisture content of the hollow fiber membrane to its weight was 3.7 mass %; the relative humidity around the hollow fiber membrane was 45%RH (25°C); and the oxygen concentration in the package was 0.08%. As a result of the evaluation of the resultant blood purifier, the amount of the eluted component from the hollow fiber membrane was 7 ppm; the amount of the consumed aqueous potassium permanganate solution was 4 mL per 1 $m^2$ of the inner surface of the hollow fiber membrane; and the amount of the eluted hydrogen peroxide found after the storage of the blood purifier under an atmosphere of 25°C and 50%RH for 3 months was 7 ppm. The results of other analyses are shown in Table 1.

(Example 6)

A bundle of hollow fiber membranes was obtained from the same spinning solution under the same spinning conditions as those in Example 1. A blood purifier which was the same as that of Example 1 was made up of the bundle of these hollow fiber membranes and was then subjected to a leak test. As a result, no failure in adhesion attributed to the sticking of the hollow fiber membranes was observed. The blood purifier was subjected to a subsequent analysis without crosslinking the hydrophilic polymer. The hollow fiber membranes were cut out of the blood purifier which had not been exposed to γ-ray, and subjected to an elution test. As a result, the amount of the eluted PVP was 6 ppm which was a sufficient level. The hollow fiber membranes were removed from the blood purifier, and the outer surfaces of the hollow fiber membranes were observed with a microscope. As a result, no defect such as flaws or the like was observed. In a blood leak test using bovine blood, no leakage of blood cells was observed. In an endotoxin permeation test, the amount of endotoxin filtered from the outside of the hollow fiber membranes to the inside thereof was smaller than the limit of detection, which was in the level of no problem.

The blood purifier and Ageless(R) Z-200PT (manufactured by Mitsubishi Gas Chemical Company, Inc.) were put in a package having an oxygen permeability of $0.5\,cm^3/(m^2.24hr.atm)$ (20°C, 90%RH) and a water vapor permeability of $3\,g/(m^2.24hr.atm)$ (40°C, 90%RH), and the inner atmosphere of the package was replaced with a nitrogen gas. After that, the package was sealed and was left to stand at a room temperature for 36 hours, and was then exposed to γ-ray to 25 kGy for sterilizing the blood purifier. The moisture content of the hollow fiber membrane to its weight was 0.3 mass %; the relative humidity around the hollow fiber membrane was 80%RH (25°C); and the oxygen concentration in the package was 0.04%. As a result of the evaluation of the resultant blood purifier, the amount of the eluted component from the hollow fiber membrane was 6 ppm; the amount of the consumed aqueous potassium permanganate solution was 2 mL per 1 $m^2$ of the inner surface of the hollow fiber membrane; and the amount of the eluted hydrogen peroxide found after the storage of the blood purifier under an atmosphere of 25°C and 50%RH for 3 months was 4 ppm. The results of other analyses are shown in Table 1.

(Comparative Example 6)

A bundle of hollow fiber membranes was obtained from the same spinning solution under the same spinning conditions as those in Example 1. A blood purifier which was the same as that of Example 1 was made up of the bundle of these hollow fiber membranes and was then subjected to a leak test. As a result, no failure in adhesion attributed to the sticking of the hollow fiber membranes was observed. The blood purifier was subjected to a subsequent analysis without crosslinking the hydrophilic polymer. The hollow fiber membranes were cut out of the blood purifier which had not been exposed to γ-ray, and subjected to an elution test. As a result, the amount of the eluted PVP was 6 ppm which was a sufficient level. The hollow fiber membranes were removed from the blood purifier, and the outer surfaces of the hollow fiber membranes were observed with a microscope. As a result, no defect such as flaws or the like was observed. In a blood leak test using bovine blood, no leakage of blood cells was observed. In an endotoxin permeation test, the amount of endotoxin filtered from the outside of the hollow fiber membranes to the inside thereof was smaller than the limit of detection, which was in the level of no problem.

The blood purifier and Ageless(R) Z-200PT (manufactured by Mitsubishi Gas Chemical Company, Inc.) were put in a package having an oxygen permeability of 0.5 $cm^3/(m^2.24hr.atm)$ (20°C, 90%RH) and a water vapor permeability of 3 $g/(m^2.24hr.atm)$ (40°C, 90%RH), and the inner atmosphere of the package was replaced with a nitrogen gas. After that, the package was sealed and was left to stand at a room temperature for 36 hours, and was then exposed to γ-ray to 25 kGy for sterilizing the blood purifier. The moisture content of the hollow fiber membrane to its weight was 2.2 mass %; the relative humidity around the hollow fiber membrane was 35%RH (25°C); and the oxygen concentration in the package was 0.03%. As a result of the evaluation of the resultant blood purifier, the amount of the eluted component from the hollow fiber membrane was 15 ppm; the amount of the consumed aqueous potassium permanganate solution was 6 mL per 1 $m^2$ of the inner surface of the hollow fiber membrane; and the amount of the eluted hydrogen peroxide found after the storage of the blood purifier under an atmosphere of 25°C and 50%RH for 3 months was 13 ppm. It was supposed that the membranes were attacked by free radicals because of the high oxygen concentration. The results of other analyses are shown in Table 1.

(Comparative Example 7)

A bundle of hollow fiber membranes was obtained from the same spinning solution under the same spinning conditions as those in Example 1. A blood purifier which was the same of Example 1 was made up of the bundle of these hollow fiber membranes and was then subjected to a leak test. As a result, no failure in adhesion attributed to the sticking of the hollow fiber membranes was observed. The blood purifier was subjected to a subsequent analysis without crosslinking the hydrophilic polymer. The hollow fiber membranes were cut out of the blood purifier which had not been exposed to γ-ray, and subjected to an elution test. As a result, the amount of the eluted PVP was 6 ppm which was a sufficient level. The hollow fiber membranes were removed from the blood purifier, and the outer surfaces of the hollow fiber membranes were observed with a microscope. As a result, no defect such as flaws or the like was observed. In a blood leak test using bovine blood, no leakage of blood cells was observed. In an endotoxin permeation test, the amount of endotoxin filtered from the outside of the hollow fiber membranes to the inside thereof was smaller than the limit of detection, which was in the level of no problem.

The blood purifier and TAMOTSU(R) (manufactured by Ohji Takku K.K.) were put in a package having an oxygen permeability of 0.5 $cm^3/(m^2.24hr.atm)$ (20°C, 90%RH) and a water vapor permeability of 3 $g/(m^2.24hr.atm)$ (40°C, 90%RH), and the inner atmosphere of the package was replaced with a nitrogen gas. After that, the package was sealed and was left to stand at a room temperature for 36 hours, and was then exposed to γ-ray to 25 kGy for sterilizing the blood purifier. The moisture content of the hollow fiber membrane to its weight was 1.6 mass %; the relative humidity around the hollow fiber membrane was 30%RH (25°C) ; and the oxygen concentration in the package was 0.12%. As a result of the evaluation of the resultant blood purifier, the amount of the eluted component from the hollow fiber membrane was 18 ppm; the amount of the consumed aqueous potassium permanganate solution was 7 mL per 1 $m^2$ of the inner surface of the hollow fiber membrane; and the amount of the eluted hydrogen peroxide found after the storage of the blood purifier under an atmosphere of 25°C and 50%RH for 3 months was 22 ppm. The results of other analyses are shown in Table 1.

(Comparative Example 8)

A bundle of hollow fiber membranes was obtained from the same spinning solution under the same spinning conditions as those in Example 1. A blood purifier which was the same as that of Example 1 was made up of the bundle of these hollow fiber membranes and was then subjected to a leak test. As a result, no failure in adhesion attributed to the sticking of the hollow fiber membranes was observed. The blood purifier was subjected to a subsequent analysis without crosslinking the hydrophilic polymer. The hollow fiber membranes were cut out of the blood purifier which had not been exposed to γ-ray, and subjected to an elution test. As a result, the amount of the eluted PVP was 6 ppm which was a sufficient level. The hollow fiber membranes were removed from the blood purifier, and the outer surfaces of the hollow fiber membranes were observed with a microscope. As a result, no defect such as flaws or the like was observed. In a blood leak test using bovine blood, no leakage of blood cells was observed. In an endotoxin permeation test, the amount of endotoxin filtered from the outside of the hollow fiber membranes to the inside thereof was smaller than the limit of detection, which was in the level of no problem.

The blood purifier and Ageless(R) Z-200PT (manufactured by Mitsubishi Gas Chemical Company, Inc.) were put in a package having an oxygen permeability of 10 $cm^3/(m^2.24hr.atm)$ (20°C, 90%RH) and a water vapor permeability

of 15 g/(m$^2$.24hr.atm) (40°C, 90%RH), and the inner atmosphere of the package was replaced with a nitrogen gas. After that, the package was sealed and was left to stand at a room temperature for 36 hours, and was then exposed to γ-ray to 25 kGy for sterilizing the blood purifier. The moisture content of the hollow fiber membrane to its weight was 2.2 mass %; the relative humidity around the hollow fiber membrane was 50%RH (25°C); and the oxygen concentration in the package was 16.2%. As a result of the evaluation of the resultant blood purifier, the amount of the eluted component from the hollow fiber membrane was 226 ppm; the amount of the consumed aqueous potassium permanganate solution was 15 mL per 1 m$^2$ of the inner surface of the hollow fiber membrane; and the amount of the eluted hydrogen peroxide found after the storage of the blood purifier under an atmosphere of 25°C and 50%RH for 3 months was 511 ppm. This event was supposed to occur because the oxygen concentration in the system was not decreased, and a similar result was observed, when the sealed package of Example 2 was used, and when the standing time of the oxygen scavenger in the package was set at 8 hours. The results of other analyses are shown in Table 1.

[Table 1]

Table 1 (Part 1)

| | | Ex. 1 | Ex. 2 | Ex. 3 | Ex. 4 | Ex. 5. | Ex. 6 |
|---|---|---|---|---|---|---|---|
| Before sterilization | Water permeability (ml/ (m$^2$.hr.mmHg)) | 510 | 342 | 602 | 290 | 339 | 343 |
| | Burst pressure (MPa) | 0.6 | 0.6 | 0.7 | 0.6 | 0.6 | 0.6 |
| | Non-uniformity in thickness | 0.73 | 0.90 | 0.82 | 0.88 | 0.90 | 0.92 |
| | Number of modules permitting blood leakage | 0 | 0 | 0 | 0 | 0 | 0 |
| | Amount of eluted PVP (ppm) | 8 | 6 | 7 | 7 | 6 | 6 |
| | PVP content in outer surface (mass %) | 33 | 27 | 29 | 32 | 27 | 27 |
| | Ratio of hole areas on outer surface (%) | 21 | 19 | 13 | 24 | 19 | 19 |
| | Av. hole area on outer surface (μm$^2$) | 0.6 | 0.5 | 0.8 | 0.9 | 0.5 | 0.5 |
| | Number of stuck membranes | 0 | 0 | 0 | 0 | 0 | 0 |
| | Amount of PVP in membrane (mass %) | 4-3 | 7.3 | 8.8 | 5.2 | 7.3 | 7.3 |
| | Permeation of endotoxin | ND | ND | ND | ND | ND | ND |
| | Insoluble component | some | None | Some | Some | None | None |
| After sterilization | Water permeability (ml/ (m$^2$.hr.mmHg)) | 508 | 328 | 588 | 288 | 337 | 326 |
| | Burst preassure (MPa) | 0.6 | 0.6 | 0.7 | 0.6 | 0.6 | 0.6 |
| | Amount of eluted. PVF (ppm) | 7 | 8 | 6 | 4 | 7 | 6 |
| | PVP content in outer surface (mass %) | 33 | 27 | 29 | 32 | 27 | 27 |
| | Amount of consumed potassium permanganoate (ml/m$^2$) | 2 | 2 | 2 | 1 | 4 | 2 |
| | Amount of eluted hydrogen peroxide after 3 months (ppm) | 3 | 2 | 4 | 6 | 7 | 4 |

Table 1 (Part 2)

| | | C. Ex. 1 | C. Ex. 2 | C. Ex. 3 | C, Ex. 4 |
|---|---|---|---|---|---|
| Before sterilization | Water permeability (ml/(m$^2$.hr.mmHg)) | 498 | 526 | - | 488 |
| | Burst pressure (MPa) | 0.2 | 0.3 | - | 0.7 |
| | Non-uniformity in thickness | 0.47 | 0.41 | - | 0.72 |
| | Number of modules permitting blood leakage | 3 | 2 | - | 0 |
| | Amount of eluted PVP (ppm) | 8 | 12 | 14 | 12 |
| | PVP content in outer surface (mass %) | 51 | 52 | 57 | 44 |
| | Ratio of hole areas on outer surface (%) | 19 | 32 | 5 | 21 |
| | Av. hole area on outer surface ($\mu$m$^2$) | 0.5 | 1.2 | 0.2 | 0.2 |
| | Number of stuck membranes | 11 | 0 | 30 | 3 |
| | Amount of PVP in membrane (mass %) | 9.6 | 7.4 | 7.7 | 6.3 |
| | Permeation of endotoxin | X | X | - | X |
| | Insoluble component | Some | Some | None | None |
| After sterilization | Water permeability (ml/(m$^2$.hr.mmHg)) | 502 | - | - | - |
| | Burst pressure (MPa) | 0.2 | - | - | - |
| | Amount of eluted PVP (ppm) | 8 | - | - | - |
| | PVP content in outer surface (mass %) | 51 | - | - | - |
| | Amount of consumed potassium permanganoate (ml/m$^2$) | 3 | - | - | - |
| | Amount of eluted hydrogen peroxide after 3 months (ppm) | 3 | - | - | - |

Table 1 (Part 3)

| | | C. Ex. 5 | C. Ex. 6 | C. Ex. 7 | C. Ex. 8 |
|---|---|---|---|---|---|
| Before sterilization | Water permeability (ml/(m$^2$.hr.mmHg)) | 502 | 329 | 336 | 496 |
| | Burst pressure (Mpa) | 0.2 | 0.6 | 0.6 | 0.6 |
| | Non-uniformity in thickness | 0.43 | 0.90 | 0.91 | 0.74 |
| | Number of modules permitting blood leakage | 4 | 0 | 0 | 0 |
| | Amour of eluted PVF (ppm) | 13 | 6 | 6 | 6 |
| | PVP content in outer surface (mass %) | 48 | 27 | 27 | 34 |
| | Ratio of hole areas on outer surface (%) | 27 | 19 | 19 | 20 |
| | Av. hole area on outer surface ($\mu$m$^2$) | 0.4 | 0.5 | 0.5 | 0.7 |
| | Number of stuck membranes | 0 | 0 | 0 | 0 |
| | Amount of PVP in membrane (mass %) | 6.1 | 7.3 | 7.3 | 4.2 |
| | Permeation of endotoxin | X | ND | ND | ND |
| | Insoluble component | Some | None | None | None |
| After sterilization | Water permeability (ml/(m$^2$(hr.mmHg)) | - | 331 | 307 | 501 |
| | Burst pressure (MPa) | | 0.6 | 0.5 | 0.6 |
| | Amount of eluted PVP (ppm) | - | 15 | 18 | 7 |

(continued)

|  |  | C. Ex. 5 | C. Ex. 6 | C. Ex. 7 | C. Ex. 8 |
|---|---|---|---|---|---|
|  | PVP content in outer surface (mass %) | - | 27 | 27 | 32 |
|  | Amount of consumed potassium permanganoate (ml/m$^2$) | - | 6 | 7 | 15 |
|  | Amount of eluted hydrogen peroxide after 3 months (ppm) | - | 13 | 22 | 511 |

INDUSTRIAL APPLICABILITY

The blood purifiers of the present invention are highly reliable in safety and performance stability, and have high water permeabilities suitable for treatment of chronic renal failure. The blood purifiers of the parent invention can be used in dried states, and therefore are light in weight and have no disadvantage of being frozen, and thus, they are high performance blood purifiers easy in hand operations. The blood purifiers of the present invention simultaneously can inhibit the elution of the components which are foreign matters to human bodies, and thus are safe as medical devices.

**Claims**

1.  A highly water-permeable hollow fiber membrane type blood purifier which comprises hydrophobic polymer hollow fiber membranes each containing a hydrophilic polymer, wherein said hollow fiber membrane has a hydrophilic polymer content of 25 to 50 mass % and a ratio of hole areas of 8 to 25% at its outer surface, and has a thickness non-uniformity degree of 0.6 or more, a thickness of 10 to 60 $\mu$m and a burst pressure of 0.5 to 2 MPa, said blood purifier being **characterized in that** said blood purifier has a water permeability of 150 to 2,000 ml/m$^2$/hr/mmHg, and **in that** said blood purifier is exposed to radioactive rays on conditions that the oxygen concentration of an ambient atmosphere around the hollow fiber membranes is from 0.001% inclusive to 0.1% inclusive, and that the moisture content of the hollow fiber membrane to its weight is from 0.2 mass % inclusive to 7 mass % inclusive.

2.  The highly water permeable hollow fiber membrane type blood purifier according to claim 1, wherein said exposure to radioactive rays is conducted on said blood purifier which is sealed in a package.

3.  The highly water permeable hollow fiber membrane type blood purifier according to claim 1 or 2, wherein the inner atmosphere of said blood purifier and/or said ambient atmosphere around the hollow fiber membranes is/are of an inert gas.

4.  The highly water permeable hollow fiber membrane type blood purifier according to any one of claims 1 to 3, wherein said exposure to radioactive rays is conducted after 10 or more hours has passed since an oxygen scavenger is put in the package.

5.  The highly water permeable hollow fiber membrane type blood purifier according to claim 4, wherein said oxygen scavenger has a moisture-releasing function.

6.  The highly water permeable hollow fiber membrane type blood purifier according to any one of claims 1 to 5, wherein said exposure to radioactive rays is conducted under the package's inner atmosphere of higher than 40%RH at 25°C.

7.  The highly water permeable hollow fiber membrane type blood purifier according to any one of claims 1 to 6, wherein the amount of an aqueous solution of potassium permanganate (2.0 X 10$^{-3}$ mol/L) consumed for the titration of an eluted component in 10 mL of an initial washing liquid for the blood purifier after the exposure to radioactive rays is 5 mL or less per 1 m$^2$ of the inner surface of the hollow fiber membrane.

8.  The highly water permeable hollow fiber membrane type blood purifier according to any one of claims 1 to 7, wherein the amount of hydrogen peroxide extracted from the hollow fiber membrane removed from the blood purifier after 3 months has passed since the exposure to radioactive rays is 10 ppm or less.

9.  The highly water permeable hollow fiber membrane type blood purifier according to any one of claims 1 to 8, wherein the oxygen permeability of said package is 1 cm$^3$/(m$^2$.24 hr.atm) or less (20°C, 90%RH).

10. The highly water permeable hollow fiber membrane type blood purifier according to any one of claims 1 to 9, wherein the water vapor permeability of said package is 5 g/(m$^2$.24 hr.atm) or less (40°C, 90%RH).

11. The highly water permeable hollow fiber membrane type blood purifier according to any one of claims 1 to 10, wherein the content of the hydrophilic polymer to the total of the hydrophobic polymer and the hydrophilic polymer is from 1 to 20 mass %.

12. The highly water permeable hollow fiber membrane type blood purifier according to any one of claims 1 to 11, wherein said hydrophobic polymer is a polysulfone-based polymer.

13. The highly water permeable hollow fiber membrane type blood purifier according to any one of claims 1 to 12, wherein said hydrophilic polymer is polyvinyl pyrrolidone.

14. The highly water permeable hollow fiber membrane type blood purifier according to any one of claims 1 to 13, wherein said hydrophilic polymer is crosslinked and insolubilized.

15. A process for manufacturing a highly water permeable hollow fiber membrane type blood purifier which comprises hydrophobic polymer hollow fiber membranes each containing a hydrophilic polymer, wherein said hollow fiber membrane has a hydrophilic polymer content of 25 to 50 mass % and a ratio of hole areas of 8 to 25% at its outer surface, and has a thickness non-uniformity degree of 0.6 or more, a thickness of 10 to 60 μm and a burst pressure of 0.5 to 2 MPa, said process being **characterized by** including a step of exposing said blood purifier having a water permeability of 150 to 2, 000 ml/m$^2$/hr/mmHg, to radioactive rays, on conditions that the oxygen concentration of an ambient atmosphere around the hollow fiber membranes is from 0.001% inclusive to 0.1% inclusive, and that the moisture content of the hollow fiber membrane to its weight is from 0.2 mass % inclusive to 7 mass % inclusive.

16. The process according to claim 15, wherein said exposure, to radioactive rays is conducted on said blood purifier which is sealed in a package.

17. The process according to claim 15 or 16, wherein said exposure to radioactive rays is conducted after 10 or more hours has passed since an oxygen scavenger is put in the package.

18. The process according to any one of claims 15 to 17, wherein said exposure to radioactive rays is conducted under the package's inner atmosphere of higher than 40%RH at 25°C.

19. The process according to any one of claims 15 to 18, wherein the amount of hydrogen peroxide extracted from the hollow fiber membrane removed from the blood purifier after 3 months has passed since the exposure to radioactive rays is 10 ppm, or less.

20. The process according to any one of claims 15 to 19, wherein the content of the hydrophilic polymer to the total of the hydrophobic polymer and the hydrophilic polymer is from 1 to 20 mass %.

<table>
<tr><td colspan="2">INTERNATIONAL SEARCH REPORT</td><td>International application No.<br>PCT/JP2005/014568</td></tr>
</table>

A.  CLASSIFICATION OF SUBJECT MATTER
    Int.Cl⁷  A61M1/18, B01D69/08, 71/68

According to International Patent Classification (IPC) or to both national classification and IPC

B.  FIELDS SEARCHED

Minimum documentation searched (classification system followed by classification symbols)
    Int.Cl⁷  A61M1/14-1/18, B01D61/00-71/68

Documentation searched other than minimum documentation to the extent that such documents are included in the fields searched
    Jitsuyo Shinan Koho          1922-1996   Jitsuyo Shinan Toroku Koho   1996-2005
    Kokai Jitsuyo Shinan Koho    1971-2005   Toroku Jitsuyo Shinan Koho   1994-2005

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| Y | JP 2688564 B2  (Daicel Chemical Industries, Ltd.),<br>10 December, 1997 (10.12.97),<br>Par. No. [0006]; table 2<br>(Family: none) | 1-20 |
| Y | JP 3212313 B2  (Teijin Ltd.),<br>25 September, 2001 (25.09.01),<br>Page 4, right column<br>(Family: none) | 1-20 |
| Y | JP 3312838 B2  (NOK Corp.),<br>12 August, 2002 (12.08.02),<br>Par. Nos. [0009] to [0011], [0028] to [0042];<br>table 1<br>(Family: none) | 1-20 |

☒  Further documents are listed in the continuation of Box C.          ☐  See patent family annex.

| | |
|---|---|
| *  Special categories of cited documents: | "T"  later document published after the international filing date or priority date and not in conflict with the application but cited to understand the principle or theory underlying the invention |
| "A"  document defining the general state of the art which is not considered to be of particular relevance | |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search<br>    28 September, 2005 (28.09.05) | Date of mailing of the international search report<br>    18 October, 2005 (18.10.05) |
|---|---|
| Name and mailing address of the ISA/<br>    Japanese Patent Office | Authorized officer |
| Facsimile No. | Telephone No. |

Form PCT/ISA/210 (second sheet) (January 2004)

| | INTERNATIONAL SEARCH REPORT | International application No. |
|---|---|---|
| | | PCT/JP2005/014568 |

C (Continuation). DOCUMENTS CONSIDERED TO BE RELEVANT

| Category* | Citation of document, with indication, where appropriate, of the relevant passages | Relevant to claim No. |
|---|---|---|
| Y | WO 1998/058842 A1 (GAMBRO KABUSHIKI KAISHA), 30 December, 1998 (30.12.98), Full text & AU 8036098 A | 1-20 |
| Y | JP 2003-245526 A (Toray Industries, Inc.), 02 September, 2003 (02.09.03), Par. No. [0042] (Family: none) | 1-20 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000107577 A
- JP 2000254222 A
- JP 2001190934 A
- JP 3193262 B
- JP 2001038170 A
- JP 2000140589 A
- JP 2001170171 A
- JP 55023620 B

- JP 8168524 A
- JP 2000288085 A
- JP 2001205057 A
- JP 62074364 A
- JP 62204754 A
- WO 9858842 A
- JP 2001170167 A
- JP 2003245526 A